# EUROPEAN PATENT SPECIFICATION

(11) **EP 2 214 485 B1**
(45) Date of publication and mention of the grant of the patent: **20.07.2016**
(21) Application number: 08833315.8
(22) Date of filing: 25.09.2008
(51) Int. Cl.: A01N 43/42, A61K 31/44, A61K 31/437, A61P 35/00

(54) **METHODS FOR TREATMENT OF CANCER**
VERFAHREN FÜR DIE BEHANDLUNG VON KREBS
MÉTHODES DE TRAITEMENT DU CANCER

(30) Priority: 25.09.2007 US 975156 P
(43) Date of publication of application: 11.08.2010
(60) Divisional application: 15197328.6
(73) Proprietor: Minerva Biotechnologies Corp., Waltham, MA 02451 (US); Bamdad, Cynthia, Waltham, MA 02451 (US)
(72) Inventor: BAMDAD, Cynthia, Waltham, MA 02451 (US)
(74) Representative: Russell, Tim
(86) International application number: PCT/US2008/077756
(87) International publication number: WO 2009/042815

(56) References cited:
- WO-A1-2007/053135
- WO-A2-03/020279
- WO-A2-2005/070930
- CN-A- 101 020 688
- US-A- 5 314 908
- US-A1- 2005 272 759
- W. WEI ET AL: 'Electrochemiluminescent detection of Mucin 1 protein and MCF-7 cancer cells based on the resonance energy transfer' THE ANALYST vol. 137, no. 9, 01 January 2012, page 2101, XP055073539 DOI: 10.1039/c2an35059a ISSN: 0003-2654
- K. ENGELMANN ET AL: 'MCF7 Side Population Cells with Characteristics of Cancer Stem/Progenitor Cells Express the Tumor Antigen MUC1' CANCER RESEARCH vol. 68, no. 7, 01 April 2008, pages 2419 - 2426, XP055073534 DOI: 10.1158/0008-5472.CAN-07-2249 ISSN: 0008-5472

## Description

### CROSS REFERENCE TO RELATED APPLICATIONS

The present application is a continuation-in-part application of U.S. Patent Application Ser. No. 09/996,069, filed November 27, 2001, which claims the benefit of priority to U.S. Provisional Patent Application Ser. Nos. 60/253,361, filed November 27, 2000; 60/256,027, filed December 15, 2000; 60/258,157, filed December 22, 2000; 60/259,615, filed January 3, 2001; 60/260,186, January 5, 2001; 60/266,169, filed February 2, 2001; 60/289,444, filed May 7, 2001; 60/266,929, filed February 6, 2001; 60/278,093, filed March 23, 2001; 60/294,887, filed May 31, 2001; and 60/298,272, filed June 14, 2001.

The present application is also a continuation-in-part application of U. S. Patent Application No. 10/237,150, filed September 5, 2002, which claims the benefit of priority to U.S. Provisional Patent Application Ser. Nos. 60/317,302, filed September 5, 2001 and 60/376,732, filed May 1, 2002.

The present application is also a continuation-in-part application of U. S. Patent Application Ser. No. 10/236,863, filed September 5, 2002, which claims the benefit of priority to U.S. Provisional Patent Application No. 60/317,302, filed September 5, 2001. The present application is also a continuation-in-part application of PCT/US2004/027954, filed August 26, 2004, which claims the benefit of priority to U.S. Provisional Patent Application No. 60/498,260, filed August 2, 2003.

The present application is also a continuation-in-part application of U. S. Patent Application Ser. No. 10/564,981, filed January 13, 2006, which claims the benefit of priority to U.S. Provisional Patent Application No. 60/610,038, filed September 14, 2004.

### BACKGROUND OF THE INVENTION

### Field of the Invention

This description generally relates to methods and compositions for the treatment of cancers that are characterized by the presence of the MUC1 receptor and, in particular, to cancers that are characterized by the aberrant expression of the MUC1 receptor.

### Description of the Related Art

All of the background information regarding the present application can be found in US Patent Application No. 10/564,981, to Bamdad.

As our knowledge of cancer grows, it has become increasingly clear that cancer is not a single disease, but rather a collection of diseases that share some common characteristics. Indeed, both the treatment and the characterization of cancers are changing rapidly as causative factors are identified at the molecular level and molecular "signatures" of sub-types of cancer are discovered. The treatment of breast cancers is being increasingly designed to target specific molecular signatures that are present in that particular cancer and in that particular patient. Excised breast tumors are often tested to determine whether they present, or present elevated levels, of estrogen receptor, progesterone-receptor, or more recently the Her2/neu receptor. The characterization of tumors at the molecular level guides the physician in the choice of possible treatments for a particular patient. Therapies that are molecularly tuned to a particular patient have had a measurable impact of cancer recurrence and survival. For example, patients with estrogen-receptor positive (ER+) and/or progesterone-receptor positive (PR+) cancers are typically treated with Tamoxifen for a period of up to 5 years. Tamoxifen, an estrogen analog, works by binding to and blocking the estrogen receptor's natural estrogen docking site. The recurrence rate of the cancer is dependent on several factors, but in general, patients with cancers that are both ER+ and PR+ fare better (efficacy ∼70%) than those that are either ER+ *or* PR+ (efficacy ∼30%), or ER-/PR- (efficacy ∼10%). Herceptin is an antibody-based therapeutic that binds to and blocks Her2/neu receptor and has been shown to be effective against tumors that over-express this receptor. GLEEVEC^{®} is a drug that treats chronic myeloid leukemia (CML). The drug inhibits the tyrosine kinase BCR-ABL, which is constitutively active in this type of cancer cell and initiates a cell growth signal. Blocking BCR-ABL and intercepting the growth signal halts proliferation; the lack of cell proliferation then induces the programmed cell death called apoptosis. Because this drug works on a target molecule that is aberrant in cancer cells, it has a very high cure rate and few if any side effects. Unfortunately, the mechanism that goes awry in CML represents only a small percentage of human cancers.

However, these results demonstrate that therapies that target specific molecules that are involved in the progression of cancer are more effective than earlier therapies that simply inhibit broad-spectrum cell growth. The new generation of cancer drugs such as HERCEPTIN^{®} and GLEEVEC^{®} and others being developed are called "smart" drugs because they home in on and disable specific molecules that are involved in cancer, or more often a particular type of cancer. Thus, in order to effectively determine which therapies are best for a particular patient, the patient's cancer can be characterized at the molecular level and treatments that act on specific aberrant molecules determined by the characterization can then be administered. Failure to characterize a cancer according to molecular signatures prior to treatment could cause the patient more harm than good. For example, by treating a patient with a drug that targets a particular molecule that is aberrant in some cancers, but not the type of cancer that the patient presents with, would constitute withholding appropriate treatment from that patient.

The MUC1 receptor is aberrantly expressed in a number of cancer types. MUC1 is a transmembrane glycoprotein found on the surface of epithelial cells. It has been reported that an estimated 75% of all human solid tumors aberrantly express the MUC1 receptor, including more than 90% of breast cancers and approximately 50% of prostate cancers. Other cancers in which the MUC 1 receptor is aberrantly expressed include ovarian, colorectal, pancreatic, some lung cancers, and several others. For some time it has been known that in a healthy cell, the MUC1 receptors are clustered at the apical border, while in cancer cells, it appears to be uniformly expressed over the entire cell surface. This loss of clustering has been correlated to degree of cancer aggressiveness and patient prognosis. It is also known that the MUC1 receptor can be cleaved and shed from the cell surface. Shed receptor can be detected in the blood of healthy patients as well as breast cancer patients. Pregnant or lactating women have higher shed MUC1 levels in serum, while non-pregnant women, regardless of previous pregnancies, have shed MUC1 present in the serum, but at significantly lower levels. Elevated levels of shed MUC1 are only present in small percentage of patients with localized disease (Stage I). As a general rule, MUC1 shedding occurs more frequently as the cancer increases in stage, becoming metastatic. Tests that assess the serum levels of shed MUC1 are approved by the FDA for the detection of breast cancer recurrence in patients initially diagnosed with Stage II or III breast cancer. These tests utilize an antibody that recognizes the terminal repeat units of the MUC1 receptor. The number of tandem repeats of the MUC1 receptor varies from person to person and is not correlated to cancer. However, because a diagnostic test or tracking test must detect *elevated* levels of shed MUC1, the variable number of antibody epitopes makes it impossible to discriminate between elevated levels and increased antibody binding because that person's MUC1 contains a greater number of tandem repeat units. This variability in the number of repeat units from person to person introduces variability into the test and thus limits its utility for tracking a patient's response to therapy and prevents its use as a diagnostic. Therefore, what is needed is either an antibody that recognizes an epitope that is expressed a single time on each shed portion of the MUC1 receptor, or an antibody that recognizes an epitope that is present on shed MUC1 when cancer is present but not when MUC1 is shed in the normal state.

Proteases comprise another category of proteins that pharmaceutical companies are investigating as therapeutic targets. For example, protease inhibitors are effective treatments for HIV. Metalloproteases have been suggested as therapeutic targets of interest for a variety of conditions, including but not limited to cancers. Metzincins are a super-family of metalloproteins that includes three families of metalloproteases: MMPs, ADAMs, and ADAMTSs (ADAMS which contain one or more thrombospondin (TS) domains). These cleavage enzymes are produced as zymogens, which are not proteolytically active until a pro-peptide or pro-domain is cleaved or removed from its surface. This final processing step typically takes place at the cell surface. However, a subset of the metzincins are cleaved to generate the active enzyme in the golgi by furin or a furin-like enzyme. TIMPs (tissue inhibitors of metalloproteinases) are small proteins that bind to some metalloproteases and inhibit their proteolytic activity.

MMPs (matrix metalloproteinases) are a class of zinc-dependent endoproteases, wherein the metal is required for its activity. Six membrane-tethered MMPs, called MT-MMPs have been identified: MT1-MMP, MT2-MMP, MT3-MMP, MT4-MMP, MT5-MMP and MT6-MMP. All the MT-MMPs are processed to the proteolytically active form by furin. The MMPs were first named for their ability to degrade components of the extracellular matrix. Now, however, MMPs as well as other metalloproteases are emerging as a class of therapeutic targets for the treatment of inflammatory diseases and cancer. ADAM-17 is currently of pharmaceutical interest for the treatment of rheumatoid arthritis because it is required for the production of soluble TNFα.

Thus, there is a need to develop new and more accurate molecular signatures of cancers, and develop new therapeutic agents that act on those molecules that are specific to a type of cancer.

### SUMMARY OF THE INVENTION

In an aspect of the invention there is provided a compound comprised of the formula: 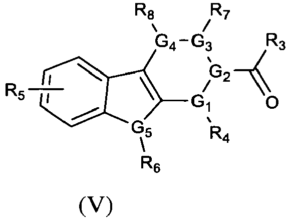 or a steroisomer, tautomer, pharmaceutically acceptable salt or ester thereof, for use in treating or preventing cancer characterised by aberrant expression of MUC1, wherein the treatment or prevention comprises a determination of whether the subject's cancer aberrantly expresses MUC1 and treating the subject with the compound if so, wherein,
R3 is selected from 2-thiofuranyl, phenyl optionally substituted with one or more of the following substituents:
   H,
   3-, or 4-Cl,
   4-F,
   2, 3, 4 ,5, 6-per-fluoro,
   2, 4-di-Cl,
   3,5-di-Cl,
   3,4-di-Cl,
   4-OCH₃,
   3-, or 4-CF₃,
   4-CN,
   2-, 3-, 4-Me,
   2, 3-di-Me,
   4-t-Bu,
   4-NO₂, and
   4-N(CH₃)₂;
R4 is phenyl ring optionally substituted with H, 4-OCH₃, 4-Cl, or 4-CF₃;
R5 is H;
R6 is H;
R7 is H;
R8 is H;
G1 is CH;
G2 is N;
G3 is CH;
G4 is CH; and
G5 is N.

Various embodiments of this aspect are as described in the dependent claims.
Subject-matter which is not encompassed by the scope of the claims does not form part of the presently claimed invention.

### BRIEF DESCRIPTION OF THE DRAWING

The present invention will become more fully understood from the detailed description given herein below, and the accompanying drawings which are given by way of illustration only, and thus are not limitative of the present invention, and wherein;
Figures 1A-1D show effects of various compounds on MUC1-positive cells (T47D) and control cells (HEK293). Delta indicates maximum difference between the cell numbers observed in control versus cancer cells.
Figure 2 is a bar graph showing the effects of various compounds on the growth of either MUC1/MUC1* positive cancer cells (T47D) or on MUC1-negative control cells (HEK293 also called K293). The amount of cell growth has been normalized wherein the amount that each cell type grew when DMSO alone is added, is defined as 100%.
Figure 3 is a bar graph showing the effects of various compounds on the growth of either MUC1/MUC1* positive cancer cells (T47D) or on MUC1-negative control cells (HEK293 also called K293). The percent cell growth is plotted.

### DETAILED DESCRIPTION OF THE INVENTION

**Definitions**

The term "MUC1 Growth Factor Receptor" (MGFR) is a functional definition meaning that portion of the MUC1 receptor that interacts with an activating ligand, such as a growth factor or a modifying enzyme such as a cleavage enzyme, to promote cell proliferation. The MGFR region of MUC1 is that extracellular portion that is closest to the cell surface and is defined by most or all by the primary sequence of MGFR (PSMGFR). The MGFR is inclusive of both unmodified peptides and peptides that have undergone enzyme modifications, such as, for example, phosphorylation, glycosylation, etc. Results of the invention are consistent with a mechanism in which this portion is made accessible to the ligand upon MUC1 cleavage at a site associated with tumorigenesis that causes release of the some or all of the IBR from the cell. MGFR is also known as MUC1*.

The term "Primary Sequence of the MUC1 Growth Factor Receptor" (PSMGFR) is a peptide sequence that defines most or all of the MGFR in some cases, and functional variants and fragments of the peptide sequence, as defined below. The PSMGFR is defined as SEQ ID NO:10 listed below in Table 1, and all functional variants and fragments thereof having any integer value of amino acid substitutions up to 20 (i.e. 1, 2, 3, 4, 5, 6, 7, 8, 9, 10, 11, 12, 13, 14, 15, 16, 17, 18, 19, or 20) and/or any integer value of amino acid additions or deletions up to 20 at its N-terminus and/or C-terminus. A "functional variant or fragment" in the above context refers to such variant or fragment having the ability to specifically bind to, or otherwise specifically interact with, ligands that specifically bind to, or otherwise specifically interact with, the peptide of SEQ ID NO:10. One example of a PSMGFR that is a functional variant of the PSMGFR peptide of SEQ NO: 10 (referred to as nat-PSMGFR - for "native") is SEQ NO: 12 (referred to as var-PSMGFR), which differs from nat-PSMGFR by including an -SPY- sequence instead of the native -SRY- (see bold text in sequence listings). Var-PSMGFR may have enhanced conformational stability, when compared to the native form, which may be important for certain applications such as for antibody production. The PSMGFR is inclusive of both unmodified peptides and peptides that have undergone enzyme modifications, such as, for example, phosphorylation, glycosylation, etc.

The term "cancer", as used herein, may include but is not limited to: biliary tract cancer; bladder cancer; brain cancer including glioblastomas and medulloblastomas; breast cancer; cervical cancer; choriocarcinoma; colon cancer; endometrial cancer; esophageal cancer; gastric cancer; hematological neoplasms including acute lymphocytic and myelogenous leukemia; multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Preferred cancers are; breast, prostate, lung, ovarian, colorectal, and brain cancer. Neoplasms in benign or malignant form are also considered within the purview of cancerous state.

The term "cancer treatment" as described herein, may include but is not limited to: chemotherapy, radiotherapy, adjuvant therapy, or any combination of the aforementioned methods. Aspects of treatment that may vary include, but are not limited to: dosages, timing of administration, or duration or therapy; and may or may not be combined with other treatments, which may also vary in dosage, timing, or duration. Another treatment for cancer is surgery, which can be utilized either alone or in combination with any of the aforementioned treatment methods. One of ordinary skill in the medical arts may determine an appropriate treatment.

As used herein, "bodily sample" refers to any body tissue or body fluid sample obtained from a subject. Preferred are body fluids, for example lymph, saliva, blood, urine, milk and breast secretions, and the like. Blood is preferred in certain embodiments. Samples of tissue and/or cells for use in the various methods described herein can be obtained through standard methods including, but not limited to: tissue biopsy, including punch biopsy and cell scraping, needle biopsy, and collection of blood or other bodily fluids by aspiration or other methods.

A "subject", as used herein, refers to any mammal (preferably, a human), and preferably a mammal that has a disease that may be treated by administering the inventive composition to a site within the subject. Examples include a human, non-human primate, cow, horse, pig, sheep, goat, dog, or cat. Generally, the invention is directed toward use with humans.

As used herein, a "MUC1-positive cancer" or a "MUC1*-positive cancer" refers to a cancer that is characterized by the aberrant expression of MUC1, wherein aberrant may refer to the overexpression of the MUC1 gene or gene product, or the loss of the normal expression pattern of MUC1 or MUC1* which, in the healthy state, is restricted to the apical border of the cell or the luminal edge of a duct or an increase in the amount of MUC1 that is cleaved and shed from the cell surface.

"Alkyl" refers to alkyl groups that do not contain heteroatoms. Thus the phrase includes straight chain alkyl groups such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example: -CH(CH₃)₂, -H(CH₃)(CH₂CH₃), -CH(CH₂CH₃)₂, -C(CH₃)₃, -C(CH₂CH₃)₃, -CH₂CH(CH₃)₂, CH₂CH(CH₃)(CH₂CH₃), -CH₂CH(CH₂CH₃)₂, -CH₂C(CH₃)₃, -CH₂C(CH₂CH₃)₃, -CH(CH₃),-CH(CH₃)(CH₂CH₃), -CH₂CH₂CH(CH₃)₂, -CH₂CH₂CH(CH₃)(CH₂CH₃), -CH₂CH₂CH(CH₂CH₃)₂,-CH₂CH₂C(CH₃)₃, -CH₂CH₂C(CH₂CH₃)₃, -CH(CH₃)CH₂-CH(CH₃)₂, -CH(CH₃)CH(CH₃)CH(CH₃)₂,-CH(CH₂CH₃)CH(CH₃)CH(CH₃)(CH₂CH₃), and others. Thus the phrase "alkyl groups" includes primary alkyl groups, secondary alkyl groups, and tertiary alkyl groups. Preferred alkyl groups include straight and branched chain alkyl groups having 1 to 12 carbon atoms.

"Alkylene" refers to the same residues as noted above for "alkyl," but having two points of attachment. Exemplary alkylene groups include ethylene (-CH₂CH₂-), propylene (-CH₂CH₂CH₂-)-, dimethylpropylene (-CH₂C(CH₃)₂CH₂-), and cyclohexylpropylene (-CH₂CH₂CH(C₆H₁₃)-).

"Alkenyl" refers to straight chain, branched, or cyclic radicals having one or more carbon-carbon double bonds and from 2 to about 20 carbon atoms. Preferred alkenyl groups include straight chain and branched alkenyl groups and cyclic alkenyl groups having 2 to 12 carbon atoms.

Alkyl or alkenyl groups may be substituted. "Substituted alkyl" refers to an alkyl group as defined above in which one or more bonds to a carbon(s) or hydrogen(s) are replaced by a bond to non-hydrogen and non-carbon atoms such as, but not limited to, a halogen atom such as F, Cl, Br, and I; an oxygen atom in groups such as hydroxyl groups, alkoxy groups, aryloxy groups, and ester groups; a sulfur atom in groups such as thiol groups, alkyl and aryl sulfide groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as in trialkylsilyl groups, dialkylarylsilyl groups, alkyldiarylsilyl groups, and triarylsilyl groups; and other heteroatoms in various other groups. Substituted alkyl groups also include groups in which one or more bonds to a carbon(s) or hydrogen(s) atom is replaced by a higher-order bond (e.g., a double- or triple-bond) to a heteroatom such as oxygen in oxo, carbonyl, carboxyl, and ester groups; nitrogen in groups such as imines, oximes, hydrazones, and nitriles. Substituted alkyl groups further include alkyl groups in which one or more bonds to a carbon(s) or hydrogen(s) atoms is replaced by a bond to an aryl, heteroaryl, heterocyclyl, or cycloalkyl group. Preferred substituted alkyl groups include, among others, alkyl groups in which one or more bonds to a carbon or hydrogen atom is/are replaced by one or more bonds to fluoro, chloro, or bromo group. Another preferred substituted alkyl group is the trifluoromethyl group and other alkyl groups that contain the trifluoromethyl group. Other preferred substituted alkyl groups include those in which one or more bonds to a carbon or hydrogen atom is replaced by a bond to an oxygen atom such that the substituted alkyl group contains a hydroxyl, alkoxy, or aryloxy group. Other preferred substituted alkyl groups include alkyl groups that have an amine, or a substituted or unsubstituted alkylamine, dialkylamine, arylamine, (alkyl)(aryl)amine, diarylamine, heterocyclylamine, diheterocyclylamine, (alkyl)(heterocyclyl)amine, or (aryl)(heterocyclyl)amine group. Still other preferred substituted alkyl groups include those in which one or more bonds to a carbon(s) or hydrogen(s) atoms is replaced by a bond to an aryl, heteroaryl, heterocyclyl, or cycloalkyl group. Examples of substituted alkyl are: -(CH₂)₃NH₂, -(CH₂)₃NH(CH₃), -(CH₂)₃NH(CH₃)₂, -CH₂C(CH₂)CH₂NH₂, -CH₂C(O)CH₂NH₂, -CH₂S(O)₂CH₃, -CH₂OCH₂NH₂,-CO₂H. Examples of substituents of substituted alkyl are: -CH₃, -C₂H₅, -CH₂OH, -OH, -OCH₃,-OC₂H₅, -OCF₃, -OC(O)CH₃, -OC(O)NH₂, -OC(O)N(CH₃)₂, -CN, -NO₂, -C(O)CH₃, -CO₂H,-CO₂CH₃, -CONH₂, -NH₂, -N(CH₃)₂, -NHSO₂CH₃ -NHCOCH₃, -NHC(O)OCH₃, -NHSO₂CH₃,-SO₂CH₃ -SO₂NH₂, Halo.

As used herein, "Me" as in "4-Me" or "OMe" refers to a methyl group.

"Halogen" or "halo" refers to chloro, bromo, fluoro, and iodo groups. The term "haloalkyl" refers to an alkyl radical substituted with one or more halogen atoms. The term "haloalkoxy" refers to an alkoxy radical substituted with one or more halogen atoms.

"Cycloalkyl" refers to a mono- or polycyclic, heterocyclic or carbocyclic alkyl substituent. Representative cycloalkyl groups include cyclopropyl, cyclobutyl, cyclopentyl, cyclohexyl, cycloheptyl, and cyclooctyl and such rings substituted with straight and branched chain alkyl groups as defined above. Typical cycloalkyl substituents have from 3 to 8 backbone (i.e., ring) atoms in which each backbone atom is either carbon or a heteroatom. The term "heterocycloalkyl" refers herein to cycloalkyl substituents that have from 1 to 5, and more typically from 1 to 4 heteroatoms in the ring structure. Suitable heteroatoms employed in compounds of the present invention are nitrogen, oxygen, and sulfur. Representative heterocycloalkyl moieties include, for example, morpholino, piperazinyl, piperadinyl, and the like. Carbocycloalkyl groups are cycloalkyl groups in which all ring atoms are carbon. When used in connection with cycloalkyl substituents, the term "polycyclic" refers herein to fused and non-fused alkyl cyclic structures.

"Substituted heterocycle," "heterocyclic group," "heterocycle," or "heterocyclyl," as used herein refers to any 3- or 4-membered ring containing a heteroatom selected from nitrogen, oxygen, and sulfur or a 5- or 6-membered ring containing from one to three heteroatoms selected from the group consisting of nitrogen, oxygen, or sulfur; wherein the 5-membered ring has 0-2 double bonds and the 6-membered ring has 0-3 double bonds; wherein the nitrogen and sulfur atom may be optionally oxidized; wherein the nitrogen and sulfur heteroatoms may be optionally quarternized; and including any bicyclic group in which any of the above heterocyclic rings is fused to a benzene ring or another 5- or 6-membered heterocyclic ring independently defined above. The term "heterocycle" thus includes rings in which nitrogen is the heteroatom as well as partially and fully-saturated rings. Preferred heterocycles include, for example: diazapinyl, pyrryl, pyrrolinyl, pyrrolidinyl, pyrazolyl, pyrazolinyl, pyrazolidinyl, imidazoyl, imidazolinyl, imidazolidinyl, pyridyl, piperidinyl, pyrazinyl, piperazinyl, N-methyl piper-azinyl, azetidinyl, N-methylazetidinyl, pyrimidinyl, pyridazinyl, oxazolyl, oxazolidinyl, isoxazolyl, isoazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, triazolyl, and benzothienyl.

Heterocyclic moieties can be unsubstituted or monosubstituted or disubstituted with various substituents independently selected from hydroxy, halo, oxo (CO), alkylimino (RN, wherein R is alkyl or alkoxy group), amino, alkylamino, dialkylamino, acylaminoalkyl, alkoxy, thioalkoxy, polyalkoxy, alkyl, cycloalkyl or haloalkyl. The heterocyclic groups may be attached at various positions as will be apparent to those having skill in the organic and medicinal chemistry arts in conjunction with the disclosure herein.

"Aryl" refers to optionally substituted monocyclic and polycyclic aromatic groups having from 3 to 14 backbone carbon or hetero atoms, and includes both carbocyclic aryl groups and heterocyclic aryl groups. Carbocyclic aryl groups are aryl groups in which all ring atoms in the aromatic ring are carbon. The term "heteroaryl" refers herein to aryl groups having from 1 to 4 heteroatoms as ring atoms in an aromatic ring with the remainder of the ring atoms being carbon atoms. When used in connection with aryl substituents, the term "polycyclic aryl" refers herein to fused and non-fused cyclic structures in which at least one cyclic structure is aromatic, such as, for example, benzodioxozolo (which has a heterocyclic structure fused to a phenyl group, i.e., naphthyl, and the like. Exemplary aryl moieties employed as substituents in compounds of the present invention include phenyl, pyridyl, pyrimidinyl, thiazolyl, indolyl, imidazolyl, oxadiazolyl, tetrazolyl, pyrazinyl, triazolyl, thiophenyl, furanyl, quinolinyl, purinyl, naphthyl, benzothiazolyl, benzopyridyl, and benzimidazolyl, and the like.

"Aralkyl" or "arylalkyl" refers to an alkyl group substituted with an aryl group. Typically, aralkyl groups employed in compounds of the present invention have from 1 to 6 carbon atoms incorporated within the alkyl portion of the aralkyl group. Suitable aralkyl groups employed in compounds of the present invention include, for example, benzyl, picolyl, and the like. Representative heteroaryls include, for example, imidazolyl, pyridyl, thiazolyl, triazolyl benzimidazolyl, benzothiazolyl, and benzoxazolyl.

"Biaryl" refers to a group or substituent to which two aryl groups, which are not condensed to each other, are bound. Exemplary biaryl compounds include, for example, phenylbenzene, diphenyldiazene, 4-methylthio-1-phenylbenzene, phenoxybenzene, (2-phenylethynyl)benzene, diphenyl ketone, (4-phenylbuta-1,3-diynyl)benzene, phenylbenzylamine, (phenylmethoxy)benzene, and the like. Preferred optionally substituted biaryl groups include: 2-(phenylamino)-N-[4-(2-phenylethynyl)-phenyl]acet- amide, 1,4-diphenylbenzene, N-[4-(2-phenylethynyl)phenyl]-2-[benzyl-amino]-acetamide, 2-amino-N-[4-(2-phenylethynyl)phenyl]propanamide, 2-amino-N-[4-(2-phenyl-ethynyl)phenyl]acetamide, 2-(cyclopropylamino)-N-[- 4-(2-phenylethynyl)-phenyl]-acetamide, 2-(ethylamino)-N-[4-(2-phenylethyny- 1)phenyl]acetamide, 2-[(2-methyl-propyl)amino]-N-[4-(2-phenylethynyl)pheny-1]acetamide, 5-phenyl-2H-benzo-[d]1,3-dioxolene, 2-chloro-1-methoxy-4-phen- ylbenzene, 2-[(imidazolylmethyl)-amino]-N-[4-(2-phenylethynyl)phenyl]aceta- mide, 4-phenyl-1-phenoxybenzene, N-(2-amino-ethyl)-[4-(2-phenylethynyl)phe- nyl]carboxamide, 2-{[(4-fluorophenyl)methyl]-amino}-N-[4-(2-phenylethynyl)- phenyl]acetamide, 2-{[(4-methylphenyl)methyl]amino}-N-[4-(2-phenyl-ethynyl-)phenyl]acetamide, 4-phenyl-1-(trifluoromethyl)benzene, 1-butyl-4-phenyl-benzene, 2-(cyclohexylamino)-N-[4-(2-phenylethynyl)pheny- 1]acetamide, 2-(ethyl-methyl-amino)-N-[4-(2-phenylethynyl)phenyl]acetamide- , 2-(butylamino)-N-[4-(2-phenyl-ethynyl)-phenyl]acetamide, N-[4-(2-phenylethynyl)phenyl]-2-(4-pyridylamino)-acetamide, N-[4-(2-phenylethynyl)phenyl]-2-(quinuclidin-3-ylamino)acetamide, N-[4-(2-phenyl-ethynyl)phenyl]pyrrolidin-2-ylcarboxamide, 2-amino-3-methyl-N-[4-(2-phenyl-ethynyl)-phenyl]butanamide, 4-(4-phenylbuta-1,3-diynyl)phenylamine, 2-(dimethyl-amino)-N-[4-(4-phenyl- buta-1,3-diynyl)phenyl]acetamide, 2-(ethylamino)-N-[4-(4-phenylbuta-1,3-di-ynyl)-phenyl]acetamide, 4-ethyl-1-phenylbenzene, 1-[4-(2-phenyl-ethynyl)-p- henyl]ethan-1-one, N-(1-carbamoyl-2-hydroxypropyl)[4-(4-phenylbuta-1,3-diy-nyl)-phenyl]-carbox-amide, N-[4-(2-phenylethynyl)phenyl]propanamide, 4-methoxy-phenyl phenyl ketone, phenyl-N-benzamide, (tert-butoxy)-N-[(4-phenylphenyl)-methyl]-carboxamide, 2-(3-phenyl-phenoxy)ethanehydroxamic acid, 3-phenylphenyl propanoate, 1-(4-ethoxyphenyl)-4-methoxybenzene, and [4-(2-phenylethynyl)phenyl]pyrrole.

"Optionally substituted" or "substituted" refers to the replacement of hydrogen with a monovalent or divalent radical. Suitable substitution groups include, for example, hydroxyl, nitro, amino, imino, cyano, halo, thio, sulfonyl, thioamido, amidino, imidino, oxo, oxamidino, methoxamidino, imidino, guanidino, sulfonamido, carboxyl, formyl, alkyl, haloalkyl, alkyamino, haloalkylamino, alkoxy, haloalkoxy, alkoxy-alkyl, alkylcarbonyl, aminocarbonyl, arylcarbonyl, aralkylcarbonyl, heteroarylcarbonyl, heteroaralkyl-carbonyl, alkylthio, aminoalkyl, cyanoalkyl, aryl and the like.

The substitution group can itself be substituted. The group substituted onto the substitution group can be carboxyl, halo, nitro, amino, cyano, hydroxyl, alkyl, alkoxy, aminocarbonyl, -SR, thioamido, -SO₃H, -SO₂R, or cycloalkyl, where R is typically hydrogen, hydroxyl or alkyl.

When the substituted substituent includes a straight chain group, the substitution can occur either within the chain (e.g., 2-hydroxypropyl, 2-aminobutyl, and the like) or at the chain terminus (e.g., 2-hydroxyethyl, 3-cyanopropyl, and the like). Substituted substituents can be straight chain, branched or cyclic arrangements of covalently bonded carbon or heteroatoms.

Certain of the compounds of the invention comprise asymmetrically substituted carbon atoms. Such asymmetrically substituted carbon atoms can result in the compounds of the invention comprising mixtures of stereoisomers at a particular asymmnetrically substituted carbon atom or a single stereoisomer. As a result, racemic mixtures, mixtures of diastereomers, as well as single diastereomers of the compounds of the invention are included in the present invention. The terms "S" and "R" configuration, as used herein, are as defined by the IUPAC 1974 "RECOMMENDATIONS FOR SECTION E, FUNDAMENTAL STEREOCHEMISTRY," Pure Appl. Chem. 45:13-30, 1976. The terms α and β are employed for ring positions of cyclic compounds. The α -side of the reference plane is that side on which the preferred substituent lies at the lower numbered position. Those substituents lying on the opposite side of the reference plane are assigned β descriptor. It should be noted that this usage differs from that for cyclic stereoparents, in which "α" means "below the plane" and denotes absolute configuration. The terms α and β configuration, as used herein, are as defined by the "Chemical Abstracts Index Guide," Appendix IV, paragraph 203, 1987.

As used herein, the term "pharmaceutically acceptable salts" refers to the nontoxic acid or alkaline earth metal salts of the compounds of the invention. These salts can be prepared in situ during the final isolation and purification of the compounds, or by separately reacting the base or acid functions with a suitable organic or inorganic acid or base, respectively. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2-hydroxyethanesulfonate, lactate, maleate, methanesulfonate, nicotinate, 2-napth-alenesulfonate, oxalate, pamoate, pectinate, persulfate, 3-phenylproionate, picrate, pivalate, propionate, succinate, sulfate, tartrate, thiocyanate, p-toluenesulfonate, and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, sulfuric acid and phosphoric acid and such organic acids as oxalic acid, maleic acid, methanesulfonic acid, succinic acid and citric acid. Basic addition salts can be prepared in situ during the final isolation and purification of the inventive compounds, or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine, and the like.

The term "pharmaceutically acceptable prodrugs" as used herein refers to those prodrugs of the compounds of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to yield the parent compound of the above formula, for example by hydrolysis in blood. A thorough discussion is provided in Higuchi, T., and V. Stella, "Pro-drugs as Novel Delivery Systems," A.C.S. Symposium Series 14, and in "Bioreversible Carriers in Drug Design," in Edward B. Roche (ed.), American Pharmaceutical Association, Pergamon Press, 1987..

The compounds are *useful in vitro* or *in vivo* in inhibiting the growth of cancer cells. The compounds may be used alone or in compositions together with a pharmaceutically acceptable carrier or excipient. Suitable pharmaceutically acceptable carriers or excipients include, for example, processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl- β -cyclodextrin, polyvinyl-pyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey, 1991.

Effective amounts of the compounds generally include any amount sufficient to detectably inhibit MUC1 positive activity by any of the assays described herein, by other MUC1 positive activity assays known to those having ordinary skill in the art, or by detecting an inhibition or alleviation of symptoms of cancer.

The amount of active ingredient that may be combined with the carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

A therapeutically effective dose will generally be a total daily dose administered to a host in single or divided doses may be in amounts, for example, of from 0.001 to 1000 mg/kg body weight daily and more preferred from 1.0 to 30 mg/kg body weight daily. Dosage unit compositions may contain such amounts of submultiples thereof to make up the daily dose.

The compounds may be administered orally, parenterally, sublingually, by aerosolization or inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols, which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The compositions in liposome form can contain, in addition to these compounds, stabilizers, preservatives, excipients, and the like. The preferred lipids are the phospholipids and phosphatidyl cholines (lecithins), both natural and synthetic. Methods to form liposomes are known in the art. See, for example, Prescott (ed.), "Methods in Cell Biology," Volume XIV, Academic Press, New York, 1976, p. 33 et seq.

While the compounds can be administered as the sole active pharmaceutical agent, they can also be used in combination with one or more other agents used in the treatment of cancer. Representative agents useful in combination with the compounds for the treatment of cancer include, for example, irinotecan, topotecan, gemcitabine, gleevec, herceptin, 5-fluorouracil, leucovorin, carboplatin, cisplatin, taxanes, tezacitabine, cyclophosphamide, vinca alkaloids, imatinib, anthracyclines, rituximab, trastuzumab, topoisomerase I inhibitors, as well as other cancer chemotherapeutic agents.

The above compounds to be employed in combination with the compounds described herein will be used in therapeutic amounts as indicated in the Physicians' Desk Reference (PDR) 47th Edition (1993), or such therapeutically useful amounts as would be known to one of ordinary skill in the art.

The compounds and the other anticancer agents can be administered at the recommended maximum clinical dosage or at lower doses. Dosage levels of the active compounds in the compositions may be varied so as to obtain a desired therapeutic response depending on the route of administration, severity of the disease and the response of the patient. The combination can be administered as separate compositions or as a single dosage form containing both agents. When administered as a combination, the therapeutic agents can be formulated as separate compositions, which are given at the same time or different times, or the therapeutic agents, can be given as a single composition.

In hematological cancers, such as chronic myelogenous leukemia (CML), chromosomal translocation is responsible for the constitutively activated BCR-ABL tyrosine kinase. The afflicted patients are responsive to GLEEVEC^{®}, a small molecule tyrosine kinase inhibitor, as a result of inhibition of Abl kinase activity. However, many patients with advanced stage disease respond to GLEEVEC^{®} initially, but then relapse later due to resistance-conferring mutations in the Abl kinase domain. In vitro studies have demonstrated that BCR-Av1 employs the Raf kinase pathway to elicit its effects. In addition, inhibiting more than one kinase in the same pathway provides additional protection against resistance-conferring mutations. Accordingly, the compounds are used in combination with at least one additional agent, such as GLEEVEC^{®}, in the treatment of hematological cancers, such as chronic myelogenous leukemia (CML), to reverse or prevent resistance to the at least one additional agent.

In addition, kits that include one or more of these compounds are provided. Representative kits include a compound and a package insert or other labeling including directions for treating a cellular proliferative disease by administering MUC1* inhibitory amount of the compound.

### Structure of Compounds

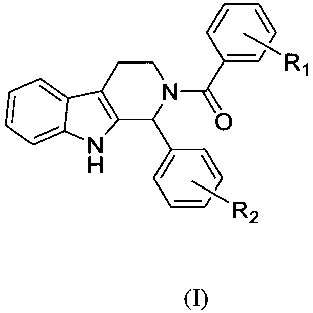

or a steroisomer, tautomer, pharmaceutically acceptable salt, ester, or prodrug thereof, wherein,
the ring to which R1 connects is optionally substituted with between 0 and 5 substituents independently selected from halogen, haloalkane, lower alkyl, branched lower alkyl, cyclic alkyl, - OH, alkylene, alkylether such as -OCH₃, -NH₂, mono- or di-substituted amine, cycloalkylamine, - NO₂, -CN, -NHCOCH₃, -NHCO-alkyl, and -NHSO₂CH₃.

The halogen may be selected from the group consisting of F, Cl, Br and I. Preferably, the halogen is Cl or F.

The haloalkane may be selected from the group consisting of CF₃ and C₂F₅, Preferably, the haloalkane is CF₃.

The lower alkyl may be selected from the group consisting of methyl, ethyl and propyl. Preferably, the lower alkyl is methyl.

The mono- or di-substituted amine may be selected from the group consisting of alkylsubstituted amine, such as but not limited to -NHCH₃ and -N(CH₃)₂, or cycloalkylamine, such as but not limited to piperidine, piperazine, morpholine, pyrrolidine.

The alkylene may be without limitation --CH=CH-CH=CH-.

The alkylether may be without limitation -OMe.
the ring to which R2 connects is optionally substituted with 0 to 5 substituents independently selected from halogen, haloalkane, lower alkyl, branched alkyl, -OH, -NH₂, mono- or di-substituted amine, cyclic amine, alkylether, and H.

The halogen may be selected from the group consisting of F, Cl, Br and I. Preferably, the halogen is Cl.

The haloalkane may be selected from the group consisting of CF₃ and C₂F₅, Preferably, the haloalkane is CF₃.

The lower alkyl may be selected from the group consisting of methyl, ethyl and propyl. Preferably, the lower alkyl is methyl.

The mono- or di-substituted amine may be selected from the group consisting of alkylsubstituted amine, such as but not limited to -NHCH₃ and -N(CH₃)₂, or cycloalkylamine, such as but not limited to piperidine, piperazine, morpholine, pyrrolidine.

The alkylether may be without limitation -OMe.

Optionally, the ring to which R1 connects is optionally substituted with between 0 and 5 substituents independently selected from F, Cl, CF₃, Me, -N(CH₃)₂, -OH, alkylene, alkylether such as -OCH₃, -NO₂, and -CN; and the ring to which R2 connects is optionally substituted with 0 to 5 substituents independently selected from -Cl, -OMe, CF₃ and H.

Optionally, the substituents may be selected from the following:
R1 may be selected from the group consisting of
   2-, 3-, or 4-Cl,
   any combination of di-chloro substitution, including but not limited to 3, 5-di-Cl,
   2-, 3-, or 4-Me,
   any combination of dimethyl including but not limited to 2, 3-di-Me,
   2-, 3-, or 4-CF₃,
   2-, 3-, 4-F,
   2, 3, 4, 5, 6-per-F,
   CH=CH-CH=CH-
   OCH₃,
   4-N(CH₃)₂,
   4-NO₂, and
   4-CN; and
R2 may be selected from the group consisting of
   4-Cl,
   4-OMe,
   4-CF₃, and
   H.

Optionally, the substituents may be selected from the following:
R1 may be selected from the group consisting of
   3- or 4-Cl
   3- or 4-Me,
   2, 3-di-Me,
   3, 5-di-Cl,
   3- or 4-CF₃,
   2, 3, 4, 5, 6-per-F,
   OCH₃,
   4-N(CH₃)₂,
   4-NO₂, and
   4-CN; and
R2 may be selected from the group consisting of
   4-Cl,
   4-OMe,
   4-CF₃, and
   H. 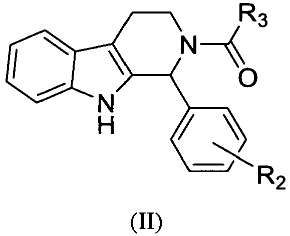
or a steroisomer, tautomer, pharmaceutically acceptable salt, ester, or prodrug thereof, wherein,
R2 is same as above.
R3 is selected from the group consisting of alkyl, -CH₂-O-cycloaryl, -CH₂-O-aryl,-CH₂NHCO-cycloalkyl, CH₂NHCO-alkyl cycloalkyl, 2-propyl, branched alkyl, thiofuranyl, aryl, heteroaryl, naphthyl, or phenyl optionally substituted with between 0 and 5 substituents independently selected from halogen, haloalkanes, lower alkyl, branched lower alkyl, cyclic alkyl,-NH₂, mono- or di-substituted amines, cycloalkylamines, -NO₂, -CN, -NHCOCH₃, -NHCO-alkyl and -NHSO₂CH₃.

The halogen may be selected from the group consisting of F, Cl, Br and I. Preferably, the halogen is Cl or F.

The haloalkane may be selected from the group consisting of CF₃ and C2F₅, Preferably, the haloalkane is CF₃.

The lower alkyl may be selected from the group consisting of methyl, ethyl propyl, and 2-propyl. Preferably, the lower alkyl is methyl or 2-propyl.

The mono- or di-substituted amine may be selected from the group consisting of alkyl substituted amine, such as but not limited to -NHCH₃ and -N(CH₃)₂, or cycloalkylamine, such as but not limited to piperidine, piperazine, morpholine, pyrrolidine.

The alkylether may be without limitation -OMe.

Optionally, the ring to which R2 connects is optionally substituted with 0 to 5 substituents independently selected from -Cl, -OMe, CF₃ and H; and R3 substituent is selected from alkyl, -CH₂-O-cycloaryl, -CH₂-O-aryl, -CH₂NHCO-cycloalkyl, CH₂NHCO-alkyl cycloalkyl, 2-propyl, branched alkyl, thiofuranyl, heteroaryl, or phenyl optionally substituted with between 0 and 5 substituents independently selected from F, Cl, CF₃, Me, -N(CH₃)₂, -NO₂, or -CN.

Optionally, the substituents may be selected from the following:
R2 may be selected from the group consisting of
   4-Cl;
   4-OMe,
   4-CF₃, and
   H.
R3 may be alkyl, cycloalkyl, 2-propyl, branched alkyl, thiofuranyl, aryl, heteroaryl, naphthyl, or phenyl optionally substituted with one or more of the following substituents:
   2-, 3-, or 4-Cl,
   CH₂NHCO-C₅H₉,
   CH₂O-C₆H₅
   any combination of di-chloro substitution, including but not limited to 3, 5-di-Cl,
   2-, 3-, or 4-Me,
   any combination of dimethyl including but not limited to 2, 3-di-Me,
   2-, 3-, or 4-CF₃,
   2-, 3-, or 4-F,
   2, 3, 4, 5, 6-per-F,
   4-N(CH₃)₂,
   4-NO₂, or
   4-CN.

Optionally, the substituents may be selected from the following:
R2 may be selected from the group consisting of
   4-Cl;
   4-OMe,
   4-CF₃, and
   H.
R3 may be cyclopentyl, 2-thiofuranyl, naphthyl, or phenyl optionally substituted with one or more of the following substituents:
   3-, or 4-Cl,
   4-F,
   2, 3, 4, 5, 6-per-fluoro,
   2, 4-di-Cl,
   3, 5-di-Cl,
   3, 4-di-Cl,
   4-OCH₃,
   CH₂NHCO-C₅H₉,
   CH₂O-C₆H₅,
   3-, or 4-CF₃,
   4-CN,
   2-, 3-, or 4-Me,
   2, 3-di-Me,
   4-t-Bu,
   4-NO₂,
   4-N(CH₃)₂, or
   H. 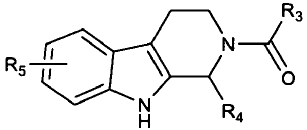 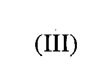
or a steroisomer, tautomer, pharmaceutically acceptable salt, ester, or prodrug thereof, wherein,
R₃ is described above;
R4 is selected from alkyl, branched alkyl, cycloalkyl, heteroaryl, or any ring or ring system optionally substituted with 0 to 5 substituents independently selected from halogen, CF₃, lower alkyl, branched alkyl, -OH, -NH₂, mono-, or di-substituted amines, including but not limited to cyclic amines such as morpholine, pyrrolidine, piperazine, piperidine, and alkylethers such as but not limited to -OMe, and H; and
R5 is selected from halogen, lower alkyl, haloalkyl, alkylether such as but not limited to - OMe, -NH₂, mono-, and di-substituted amines, including but not limited to cyclic amines such as morpholine, pyrrolidine, piperazine, and piperidine, and H.

Optionally, the substituents may be selected from the following:
R3 is selected from alkyl, cycloalkyl, 2-propyl, branched alkyl, thiofuranyl, heteroaryl, or phenyl optionally substituted with between 0 and 5 substituents independently selected from halogen, such as F, Cl, Br, I, haloalkanes, such as CF₃, lower alkyl such as methyl, ethyl, propyl, dialkyl-substituted amines such as -N(CH₃)₂, -NO₂, -CN;
R4 is selected from an aromatic ring optionally substituted with 0 to 5 substituents independently selected from halogen, lower alkyl, haloalkyl, alkylether such as -OMe, and H; and R5 is selected from halogen, lower alkyl, haloalkyl, alkylether such as -OMe, and H.

Optionally, the substituents may be selected from the following:
R3 is selected from alkyl, cycloalkyl, 2-propyl, branched alkyl, thiofuranyl, heteroaryl, or phenyl optionally substituted with between 0 and 5 substituents independently selected from F, Cl, CF₃, Me, -N(CH₃)₂, -NO₂, and -CN,
R4 is selected from aromatic ring optionally substituted with 0 to 5 substituents independently selected from halogen, alkyloxy, haloalkyl and H; and
R5 is selected from H, F, Cl, OCH₃, CH₃, CN, NO₂ or NH₂.

Optionally, the substituents may be selected from the following:
R3 is selected from alkyl, cycloalkyl, 2-propyl, branched alkyl, thiofuranyl, heteroaryl, or phenyl optionally substituted with one or more of the following substituents:
   2-, 3, or 4-Cl, any combination of di-chloro substitution, including but not limited to 3, 5-di-Cl, 2-, 3-, or 4-Me,
   any combination of dimethyl including but not limited to 2, 3-di-Me, 2-, 3-, or 4-CF₃,
   2-, 3-, or 4-F,
   2, 3, 4, 5, 6-per-F,
   4-N(CH₃)₂,
   4-NO₂,
   4-CN;
R4 is selected from phenyl ring optionally substituted with Cl, OMe, and H, CF₃; and R5 is selected from H, F, and Cl.

Optionally, the substituents may be selected from the following:
R3 is selected from cyclopentyl, 2-thiofuranyl, and phenyl optionally substituted with one or more of the following substituents:
   H,
   3-, or 4-Cl,
   4-F,
   2, 3, 4, 5, 6-per-fluoro,
   2, 4-di-Cl,
   3, 5-di-Cl,
   3, 4-di-Cl,
   4-OCH₃,
   3-, or 4-CF₃,
   4-CN,
   2-, 3-, or 4-Me,
   2, 3-di-Me,
   4-t-Bu,
   4-NO₂,
   4-N(CH₃)₂;
R4 is a phenyl ring optionally substituted with H, 4-OCH₃, 4-Cl, or 4-CF₃; and
R5 is H. 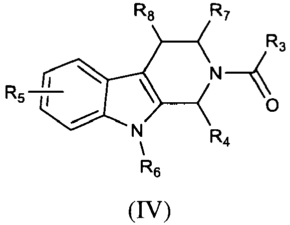
or a steroisomer, tautomer, pharmaceutically acceptable salt, ester, or prodrug thereof, wherein,
R3 is described above;
R4 is described above;
R5 is described above;
R6 is H or alkyl;
R7 is H or alkyl, -OH, -NH₂, mono-, and di-substutited amines, including cyclic amines such as morpholine, pyrrolidine, piperazine, and piperidine; and
R8 is H or alkyl.

Optionally, the substituents may be selected from the following:
R3 is selected from alkyl, cycloalkyl, 2-propyl, branched alkyl, thiofuranyl, heteroaryl, or phenyl optionally substituted with between 0 and 5 substituents independently selected from halogen, including F, Cl, Br, and I, haloalkanes, such as CF₃, lower alkyl such as methyl, ethyl, propyl, dialkyl-substituted amines such as -N(CH₃)₂, -NO₂, and -CN;
R4 is selected from an aromatic ring optionally substituted with 0 to 5 substituents independently selected from halogen, lower alkyl, haloalkyl, alkylethers such as -OMe, and H;
R5 is selected from halogen, lower alkyl, haloalkyl, alkylether such as -OMe, and H;
R6 is H or alkyl;
R7 is H, alkyl, -OH, or -NH₂; and
R8 is H or alkyl.

Optionally, the substituents may be selected from the following:
R3 is alkyl, cycloalkyl, 2-propyl, branched alkyl, thiofuranyl, heteroaryl, or phenyl optionally substituted with between 0 and 5 substituents independently selected from F, Cl, CF₃, Me, -N(CH₃)₂, -NO₂, and -CN;
R4 is aromatic ring optionally substituted with 0 to 5 substituents independently selected from halogen, alkyloxy, haloalkyl and H;
R5 is H, F, Cl, OCH₃, CH₃, CN, NO₂, or NH₂;
R6 is H or alkyl;
R7 is H or alkyl; and
R8 is H or alkyl.

Optionally, the substituents may be selected from the following:
R3 is selected from alkyl, cycloalkyl, 2-propyl, branched alkyl, thiofuranyl, heteroaryl, or phenyl optionally substituted with one or more of the following substituents:
   2-, 3-, or 4-Cl,
   any combination of di-chloro substitution, including but not limited to 3, 5-di-Cl,
   2-, 3-, or 4-Me,
   any combination of dimethyl including but not limited to 2, 3-di-Me,
   2-, 3-, or 4-CF₃,
   2-, 3-, 4-F,
   2, 3, 4, 5, 6-per-F,
   4-N(CH₃)₂,
   4-NO₂, and
   4-CN;
R4 is phenyl ring optionally substituted with Cl, OMe, H, or CF₃;
   R5 is H, F, or Cl;
   R6 is H or CH₃;
   R7 is H or CH₃; and
   R8 is H or CH₃.

Optionally, the substituents may be selected from the following:
R3 is selected from cyclopentyl, 2-thiofuranyl, phenyl optionally substituted with one or more of the following substituents:
   H,
   3-, 4-Cl,
   4-F,
   2, 3, 4, 5, 6-per-fluoro,
   2, 4-di-Cl,
   3, 5-di-Cl,
   3, 4-di-Cl,
   4-OCH₃,
   3-, 4-CF₃,
   4-CN,
   2-, 3-, 4-Me,
   2, 3-di-Me,
   4-t-Bu,
   4-NO₂, and
   4-N(CH₃)₂;
R4 is phenyl ring optionally substituted with H, 4-OCH₃, 4-Cl, or 4-CF₃;
R5 is H;
R6 is H;
R7 is H; and
R8 is H. 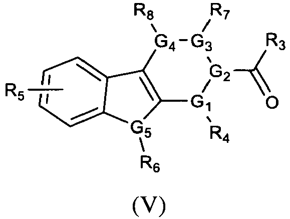
or a steroisomer, tautomer, pharmaceutically acceptable salt, ester, or prodrug thereof, wherein,
R3 is described above;
R4 is described above;
R5 is described above;
R6 is described above;
R7 is described above;
R8 is described above;
G1 is CH;
G2 is N;
G3 is CH, CH₂, NH;
G4 is CH or CH₂; and
G5 is N, O, or S.

Optionally, the substituents may be selected from the following:
R3 is selected from alkyl, cycloalkyl, 2-propyl, branched alkyl, thiofuranyl, heteroaryl, or phenyl optionally substituted with between 0 and 5 substituents independently selected from halogen, such as F, Cl, Br, I, haloalkane, such as CF₃, lower alkyl such as methyl, ethyl, propyl, dialkyl-substituted amines such as -N(CH₃)₂, -NO₂, and -CN;
R4 is selected from an aromatic ring optionally substituted with 0 to 5 substituents independently selected from halogen, lower alkyl, haloalkyl, alkylether such as -OMe, and H;
R5 is selected from halogen, lower alkyl, haloalkyl, alkylethers such as -OMe, and H;
R6 is H or alkyl;
R7 is H or alkyl, -OH, or -NH₂;
R8 is H or alkyl;
G1 is CH;
G2 is N;
G3 is CH, CH₂, or NH;
G4 is CH or CH₂; and
G5 is N, O, or S.

Optionally, the substituents may be selected from the following:
R3 is selected from alkyl, cycloalkyl, 2-propyl, branched alkyl, thiofuranyl, heteroaryl, or phenyl optionally substituted with between 0 and 5 substituents independently selected from: F, Cl, CF₃, Me, -N(CH₃)₂, -NO₂, and -CN;
R4 is aromatic ring optionally substituted with 0 to 5 substituents independently selected from halogen, alkyloxy, haloalkyl and H;
R5 is H, F, Cl, OCH₃, CH₃, CN, NO₂, or NH₂;
R6 is H or alkyl;
R7 is H or alkyl;
R8 is H or alkyl;
G1 is CH;
G2 is N;
G3 is CH, CH₂, or NH;
G4 is CH or CH₂; and
G5 is N, O or S.

Optionally, the substituents may be selected from the following:
R3 is selected from alkyl, cycloalkyl, 2-propyl, branched alkyl, thiofuranyl, heteroaryl, or phenyl optionally substituted with one or more of the following substituents:
   2-, 3-, or 4-Cl,
   any combination of di-chloro substitution, including but not limited to 3, 5-di-Cl, 2-, 3-, or 4-Me,
   any combination of dimethyl including but not limited to 2, 3-di-Me, 2-, 3-, or 4-CF₃,
   2-, 3-, or 4-F,
   2, 3, 4, 5, 6-per-F,
   4-N(CH₃)₂,
   4-NO₂,
   4-CN;
R4 is phenyl ring optionally substituted with Cl, OMe, H, or CF₃;
   R5 is H, F or Cl;
   R6 is H or CH₃;
   R7 is H or CH₃;
   R8 is H or CH₃;
   G1 is CH;
   G2 is N;
   G3 is CH or CH₂;
   G4 is CH or CH₂; and
   G5 is N.

Optionally, the substituents may be selected from the following:
R3 is selected from cyclopentyl, 2-thiofuranyl, phenyl optionally substituted with one or more of the following substituents:
   H,
   3-, or 4-Cl,
   4-F,
   2, 3, 4 ,5, 6-per-fluoro,
   2, 4-di-Cl,
   3, 5-di-Cl,
   3 ,4-di-Cl,
   4-OCH₃,
   3-, or 4-CF₃,
   4-CN,
   2-, 3-, 4-Me,
   2, 3-di-Me,
   4-t-Bu,
   4-NO₂,
   4-N(CH₃)₂;
R4 is phenyl ring optionally substituted with H, 4-OCH₃, 4-Cl, 4-CF₃;
   R5 is H;
   R6 is H;
   R7 is H;
   R8 is H;
   G1 is CH;
   G2 is N;
   G3 is CH or CH₂;
   G4 is CH or CH₂; and
   G5 is N.

### Activities of compounds having specific activity against cancer cells

### Chemotype 4 Compounds.

### Two parent compounds:

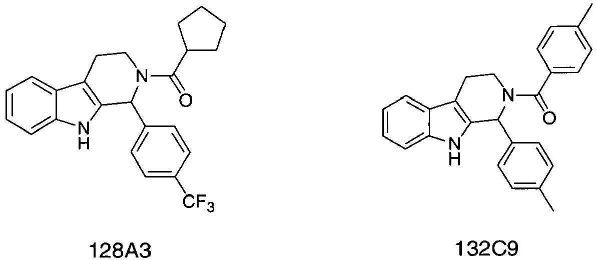

All analog compounds that were tested include the following:

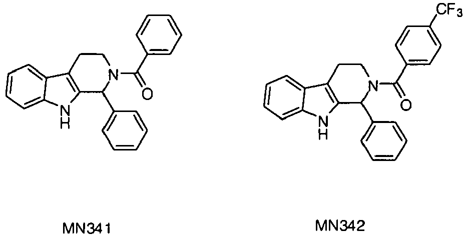

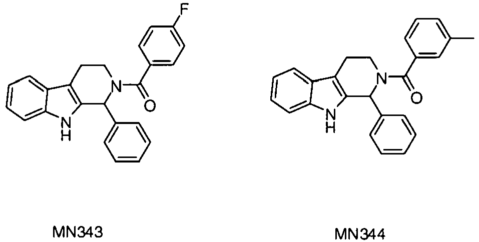

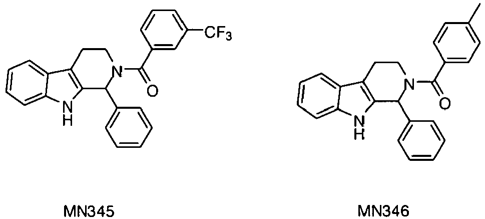

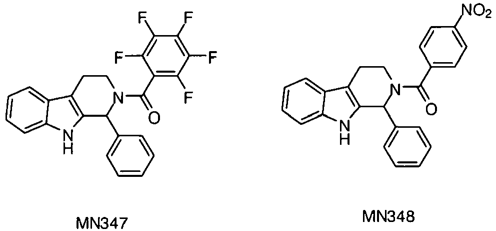

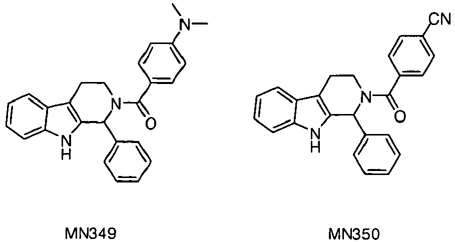

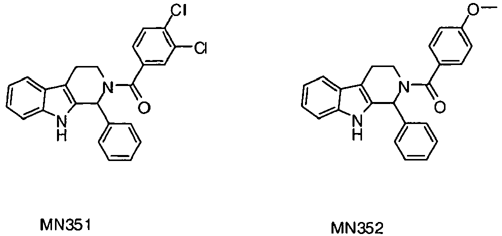

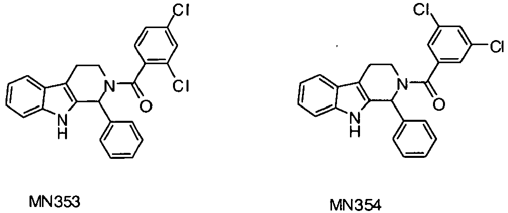

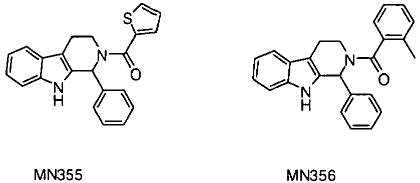

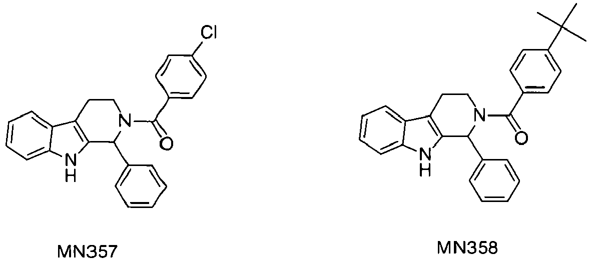

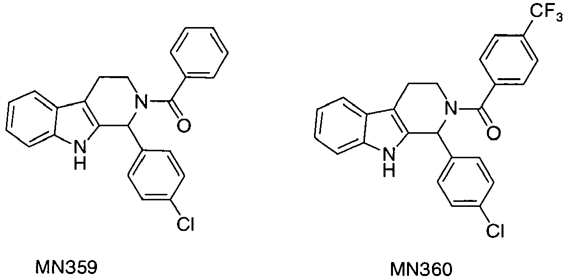

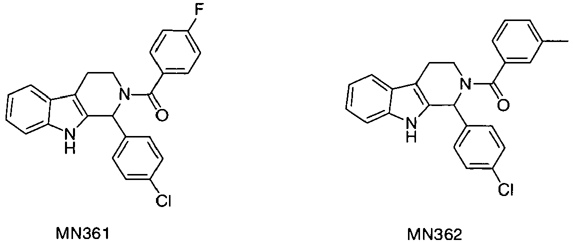

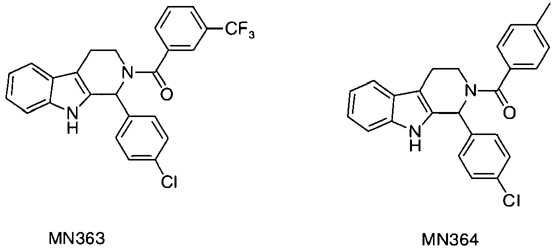

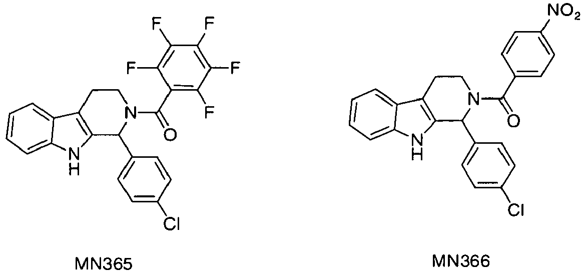

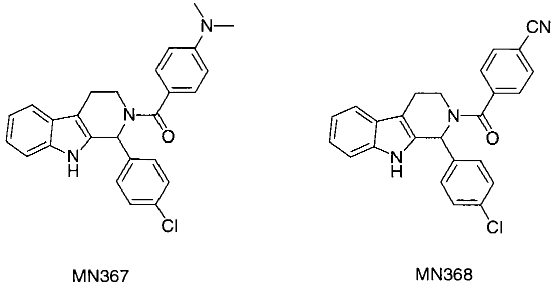

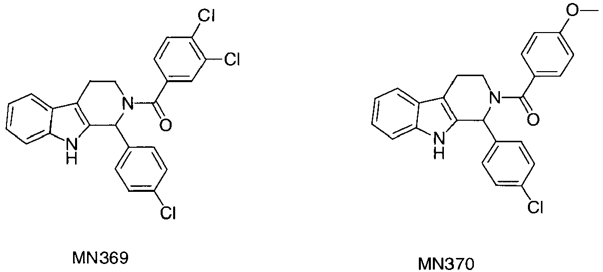

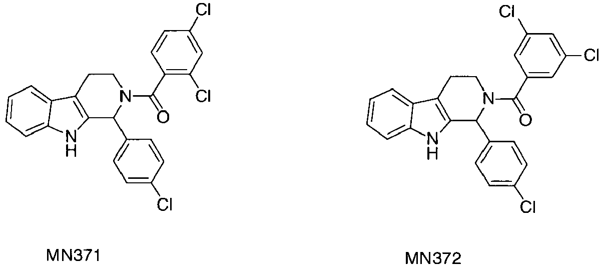

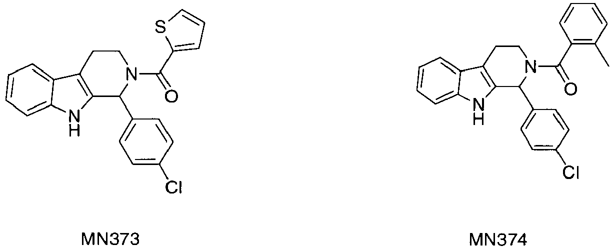

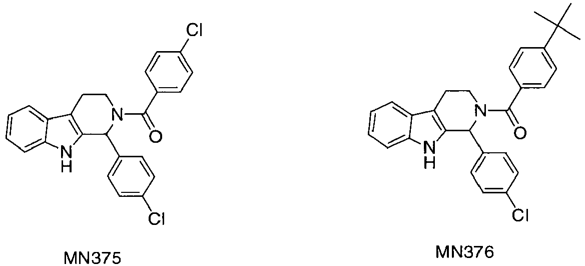

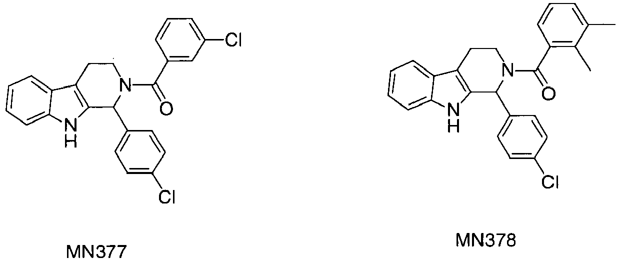

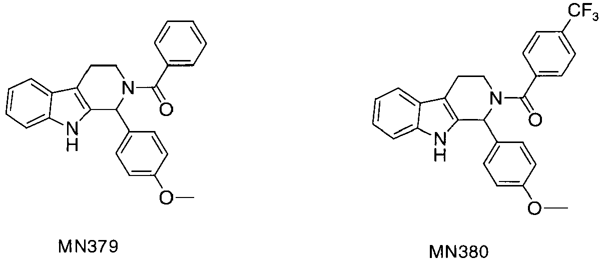

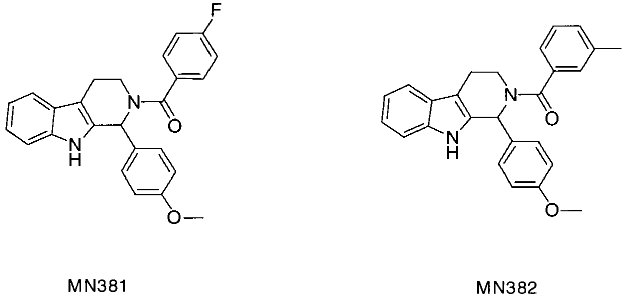

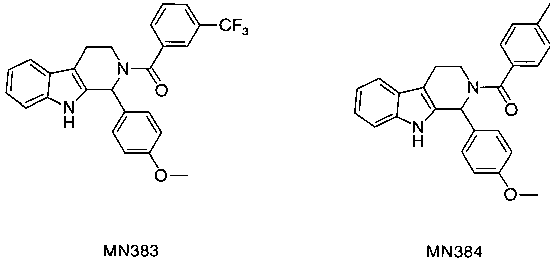

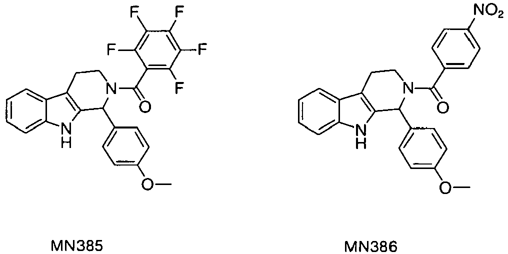

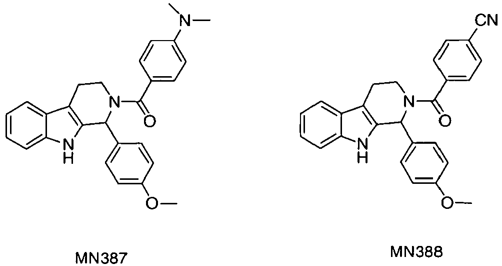

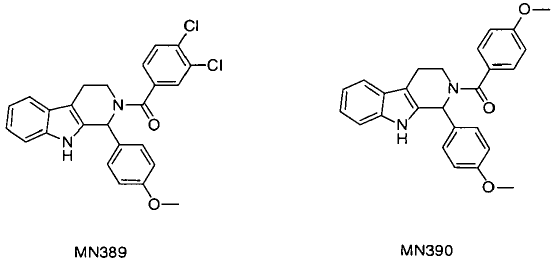

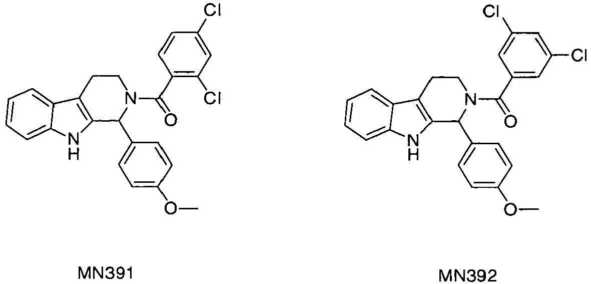

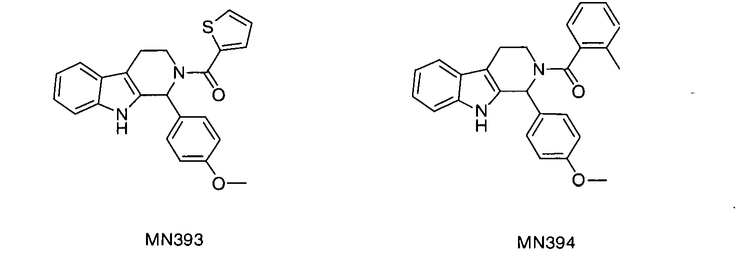

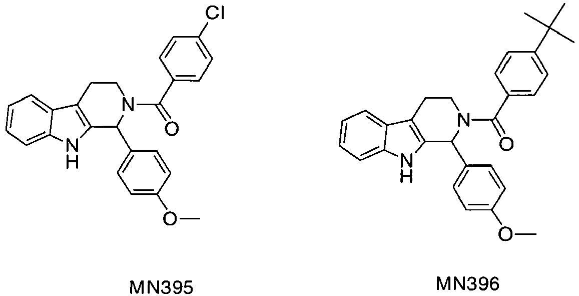

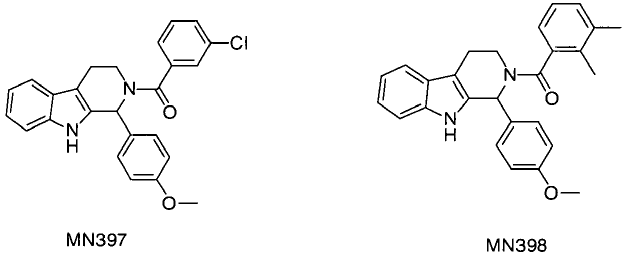

Other analog compounds:

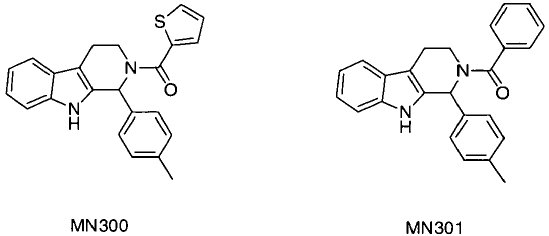

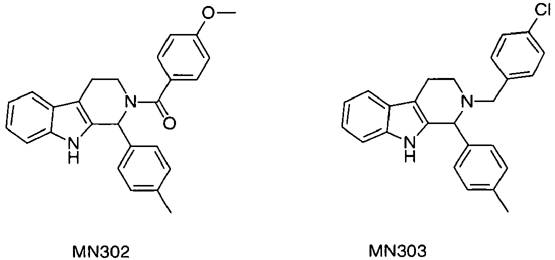

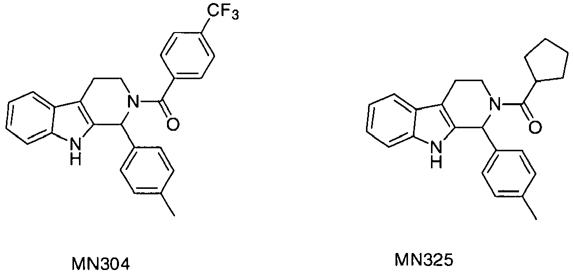

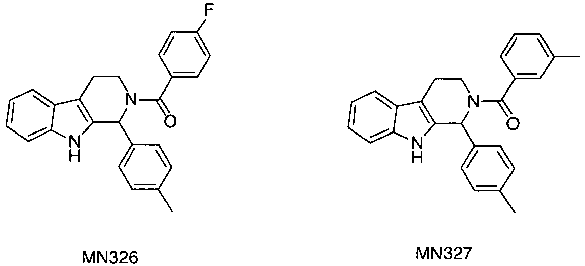

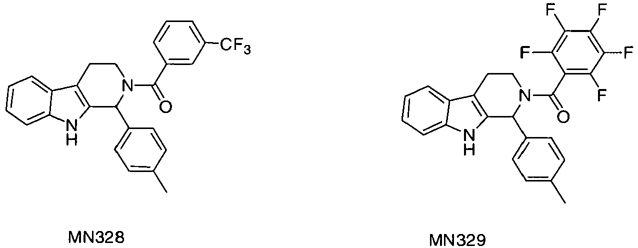

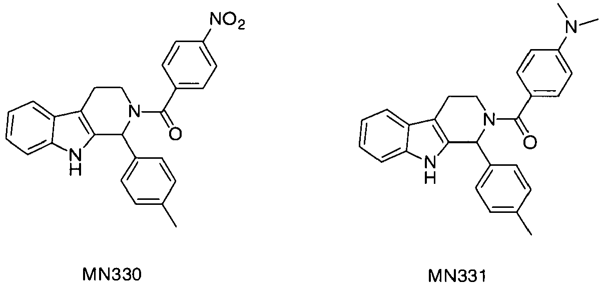

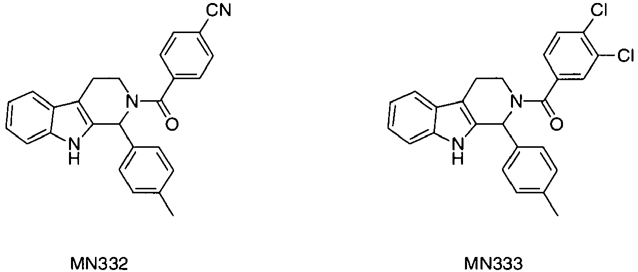

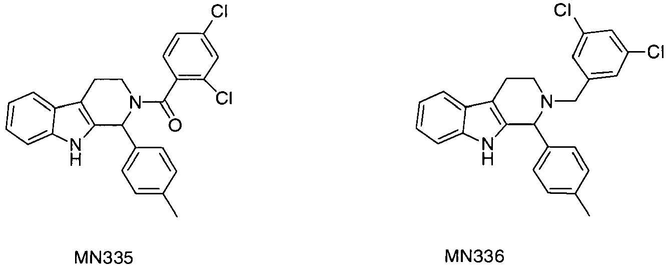

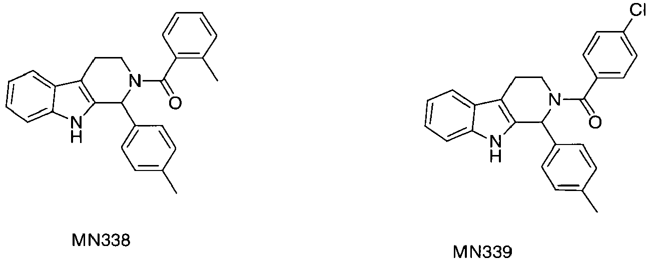

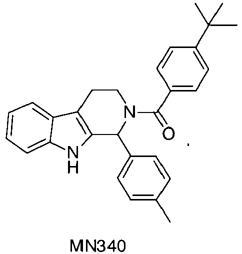

| | |
|---|---|
| CGX-0536615 | CGX-0499824 |
| 132F9 | 63B11 |
| T47D -100; K293 32; [K293-T47D] 132 | T47D -77; K293 46; [K293-T47D] 123 |
| 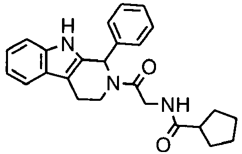 | 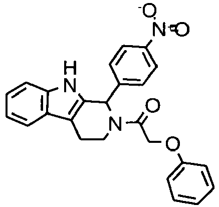 |
| MN400 | MN401 |

| | |
|---|---|
| CGX-0499826 | CGX-0500116 |
| 58G6 | 128A3 |
| T47D -100: K293 19; [K293-T47D] 119 | T47D -72; K293 45; [K293-T47D] 117 |
| 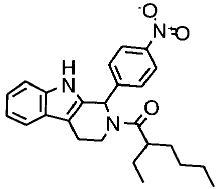 | 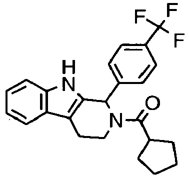 |
| MN402 | MN403/128A3 |

| | |
|---|---|
| CGX-0523850 | CGX-0494775 |
| 130A7 | 64C10 |
| T47D -90; K293 19; [K293-T47D] 109 | T47D -100; K293 -16; [K293-T47D] 84 |
| 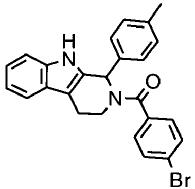 | 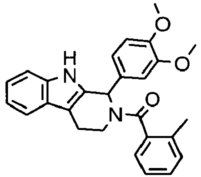 |
| MN404 | MN405 |

| | |
|---|---|
| CGX-0494750 | CGX-0499832 |
| 64D5 | 20A9 |
| T47D -100; K293 -22; [K293-T47D] 78 | T47D -35; K293 15; [K293-T47D] 50 |
| 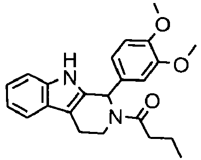 | 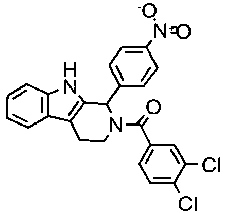 |
| MN406 | MN407 |

| | |
|---|---|
| CGX-0500078 | CGX-0499815 |
| 130 B3 | 34 H6 |
| T47D -24; K293 26; [K293-T47D] 50 | T47D -2; K293 41; [K293-T47D] 43 |
| 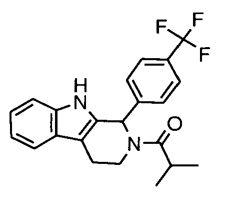 | 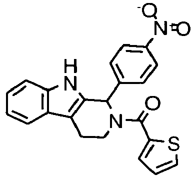 |
| MN408 | MN409 |

| | |
|---|---|
| CGX-0494729 | |
| 99 F2 | |
| T47D 35; K293 35; [K293-T47D] 0 | |
| 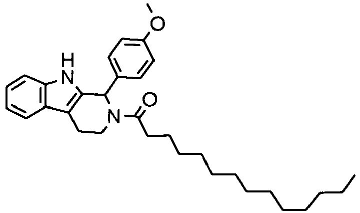 | |
| MN410 | |

### EXAMPLES

### Synthesis schemes for chemotype 4 compounds

### A. Synthesis scheme for compound 128A3

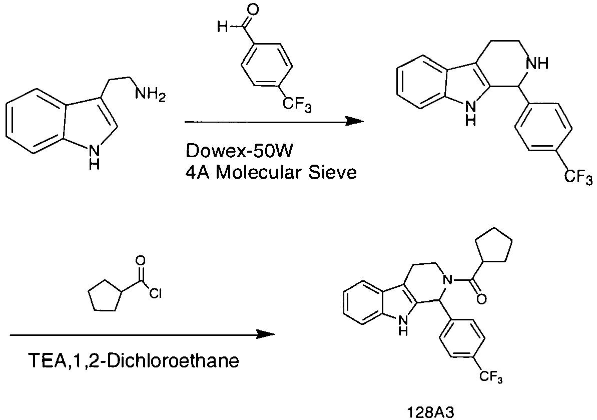

### Example 1 - Synthesis of cyclopentyl-[1-(4-trifluoromethyl-phenyl)-1,3,4,9-tetrahydro-β-carbolin-2-yl]-methanone (compound 128A3)

### Example 1.1 - 1-(4-Trifluoromethyl-phenyl)-2,3,4,9-tetrahydro-1H-β-carboline

A dry 500 ml round bottomed flask, equipped with a stir bar was charged with 5.0 g of tryptamine (1 eq.), 2.0 g of Dowex-50W, 5.0 g of 4A molecular sieves (as a dehydrating agent) and 150 ml of chloroform. 5.4 g (1 eq.) of 4-(Trifluoromethyl)benzaldehyde was added via syringe, followed by the addition of 1.0 ml of trifluoroacetic acid (0.42 eq.) The reaction mixture was gently stirred. Completion of the reaction was monitored by TLC, after which 1,2 - DCE was added to reaction mixture. Solvents were removed to give the crude title product which was used for next step without purification.

### Example 1.2 - Cyclopentyl-[1-(4-trifluoromethyl-phenyl)-1,3,4,9-tetrahydro-β-carbolin-2-yl]-methanone

A dry 500 ml round bottomed flask, equipped with a large stir bar was charged with 31.2 mmol of crude 1-(4-trifluoromethyl-phenyl)-2,3,4,9-tetrahydro-1H-β-carboline (1 eq.), 4.76 g of triethylamine (1.5 eq.) and 300 ml of 1,2-DCE. After the reaction vessel was sealed, 4.14 g (1 eq.) of cyclopentanecarbonyl chloride was added via syringe dropwise. Completion of the reaction was monitored by TLC, after which the solution was quenched with 100 ml of saturated NaHCO₃ aqueous solution and diluted with 500 ml of ethyl acetate. The organic layer was separated, washed with brine and dried with NaSO₄. After the solvent was removed, flash chromatography (3% CH₃OH / CH₂Cl₂ with 0.25% NH₄OH) allowed isolation of the title product (1.5 g, 12% for two steps) as a yellow solid.

### B. Synthesis schemes for compounds MN341 - MN358

### Compounds with phenyl substitution

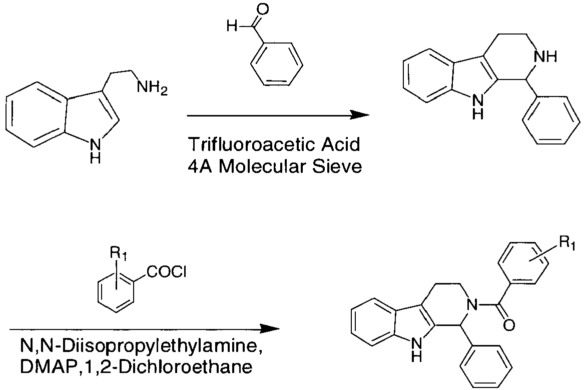

R₁ = H, *p*-CF₃, *p*-F, *m*-Me, *p*-CF₃, *p*-Me, pentyl-F, *p*-NO₂, *p*-NMe₂, *p*-CN, *p,m*-DiCl, *p*-OMe, *p,o*-DiCl, *m,m*-DiCl, *o*-Me, *p*-Cl, *p*-tBu

### Compounds with thiophene ring

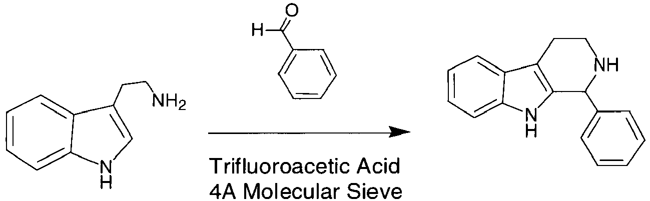

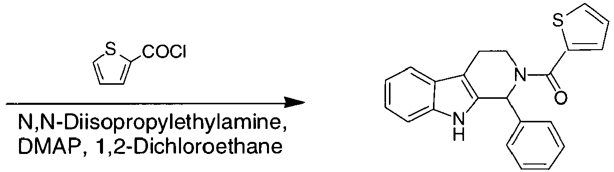

### Example 2 - Synthesis of Phenyl-(1-phenyl-1,3,4,9-tetrahydro-β-carbolin-2-yl)-methanone (compound MN341)

### Example 2.1 - 1-Phenyl-2,3,4,9-tetrahydro-1H-β-carboline

A 500 ml round bottomed flask, equipped with a condenser and oil bath was charged with 10.0 g of tryptamine (1 eq.), 6.62 g (1 eq.) of benzaldehyde, 5 g of 4A molecular sieves (as a dehydrating agent) and 200 ml of 1,2 - DCE. After dissolution, 2.85 g (0.4 eq.) of trifluoroacetic acid was added in one portion. The reaction mixture was brought to reflux. Completion of the reaction was monitored by TLC, after which the temperature was cooled to 30 °C, followed by the removal of molecular sieves through a loose glass wool plug. The solution was quenched with 150 ml of saturated NaHCO₃ aqueous solution and diluted with 500 ml of ethyl acetate. The organic layer was separated, washed with brine and dried with MgSO₄. After the solvent was removed, flash chromatography (10% CH₃OH / CH₂Cl₂) allowed isolation of the title product (3.1 g, 20%) as a solid.

### Example 2.2 - Phenyl-(1-phenyl-1,3,4,9-tetrahydro-β-carbolin-2-yl)-methanone

0.2 g of 1-Phenyl-2,3,4,9-tetrahydro-1H-β-carboline (1eq.) and 0.4 ml of N,N-diisopropylethylamine (2.8 eq.) were dissolved with 8 ml of 1,2 - DCE in a scintillation vial. 0.281 g of Benzoyl chloride (2.5 eq.) and 6.7 mg of 4-dimethylaminopyridine (0.07 eq.) were dissolved with 1 ml of 1,2 - DCE in another scintillation vial. These two solutions were mixed together, sealed and stirred overnight. Completion of the reaction was monitored by TLC, after which 2 ml of 10% citric acid aqueous solution was added to the reaction mixture. The solution was diluted with 50 ml of ethyl acetate. The organic layer was separated, washed twice with 10 ml of saturated NaHCO₃ aqueous solution, brine and dried with MgSO₄. After the solvent was removed, flash chromatography allowed isolation of the title product (0.24 g, 86%) as a solid.

### C. synthesis schemes for compounds MN359 - MN378

### Compound with phenyl substitution

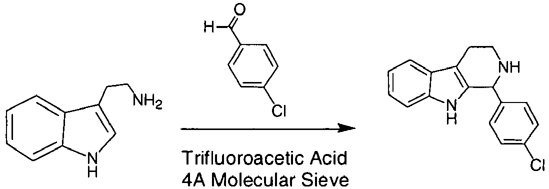

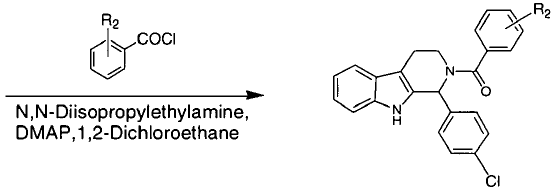

R₂ = H, *p*-CF₃, p-F, *m*-Me, *m*-CF₃, *p*-Me, pentyl-F, *p*-NO₂, *p*-NMe₂, *p*-CN, *p,m*-DiCl, *p*-OMe, *p,o*-DiCl, *m,m*-DiCl, *o*-Me, *p*-Cl, *p*-^{t}Bu, *m*-Cl, *m,o*-DiCl

### Compound with thiophene ring

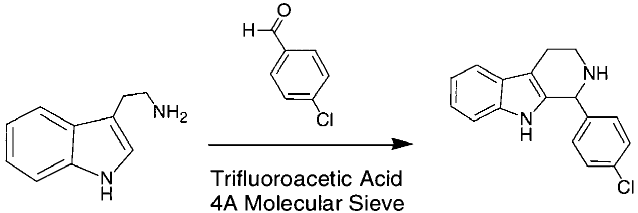

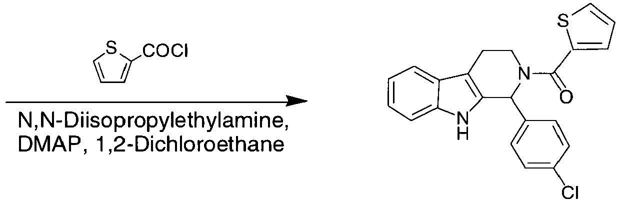

### Example 3 - [1-(4-Chloro-phenyl)-1,3,4,9-tetrahydro-β-carbolin-2-yl]-phenyl-methanone (compound MN359)

### Example 3.1 - 1-(4-Chloro-phenyl)-2,3,4,9-tetrahydro-1H-β-carboline

A 500 ml round bottomed flask, equipped with a condenser and oil bath was charged with 10.0 g of tryptamine (1 eq.), 8.77 g (1 eq.) of 4-chlorobenzaldehyde, 5 g of 4A molecular sieves (as a dehydrating agent) and 200 ml of 1,2 - DCE. After dissolution, 2.85 g (0.4 eq.) of trifluoroacetic acid was added in one portion. The reaction mixture was brought to reflux. Completion of the reaction was monitored by TLC, after which the temperature was cooled to 30 °C, followed by the removal of molecular sieves through a loose glass wool plug. The solution was quenched with 150 ml of saturated NaHCO₃ aqueous solution and diluted with 500 ml of ethyl acetate. The organic layer was separated, washed with brine and dried with MgSO₄. After the solvent was removed, flash chromatography (10% CH₃OH / CH₂Cl₂) allowed isolation of the title product (7.8 g, 32%) as a solid.

### Example 3.2 - [1-(4-Chloro-phenyl)-1,3,4,9-tetrahydro-β-carbolin-2-yl]-phenyl-methanone

0.2 g of 1-(4-Chloro-phenyl)-2,3,4,9-tetrahydro-1H-β-carboline (1eq.), 0.46 ml of N,N-diisopropylethylamine (3.7 eq.) and 23 mg of 4-dimethylaminopyridine (0.27 eq.) were dissolved with 8 ml of 1,2 - DCE in a scintillation vial. 0.28 g of Benzoyl chloride (2.8 eq.) was dissolved with 1 ml of 1,2 - DCE in another scintillation vial. These two solutions were mixed together, sealed and stirred overnight. Completion of the reaction was monitored by TLC, after which 2 ml of 10% citric acid aqueous solution was added to the reaction mixture. The solution was diluted with 50 ml of ethyl acetate. The organic layer was separated, washed twice with 10 ml of saturated NaHCO₃ aqueous solution, brine and dried with MgSO₄. After the solvent was removed, flash chromatography allowed isolation of the title product (0.24 g, 87%) as a solid.

### D. Synthesis schemes for MN379 - MN398

### Compound with phenyl substitution

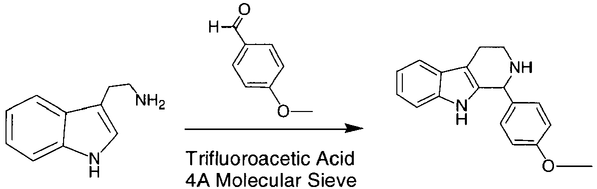

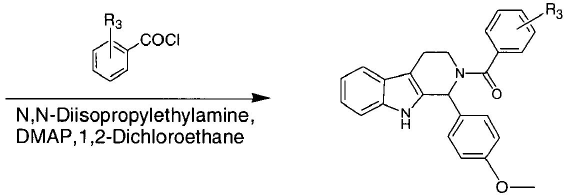

R₃ = H, *p*-CF₃, *p*-F, *m*-Me, *m*-CF₃, *p*-Me, pentyl-F, *p*-NO₂, *p*-NMe₂, *p*-CN, *p,m*-DiCl, *p*-OMe, *p,o*-DiCl, *m,m*-DiCl, *o*-Me, *p*-Cl, *p*-^{t}Bu, *m*-Cl, *m,o*-DiCl

### Compound with thiophene ring

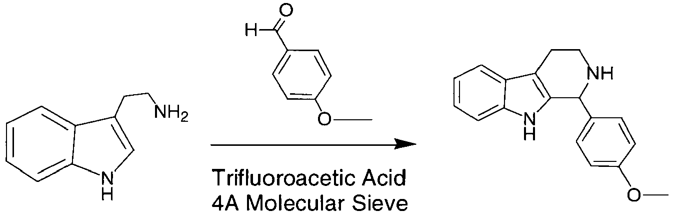

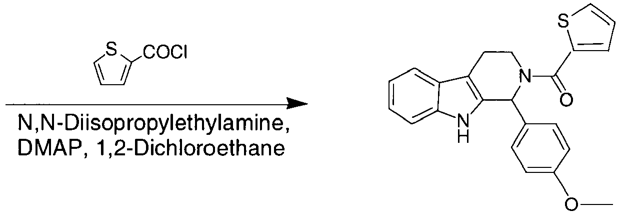

### Example 4 - [1-(4-Methoxy-phenyl)-1,3,4,9-tetrahydro-β-carbolin-2-yl]-phenyl-methanone (compound MN379)

### Example 4.1 1-(4-Methoxy-phenyl)-2,3,4,9-tetrahydro-1H-β-carboline

A 500 ml round bottomed flask, equipped with a condenser and oil bath was charged with 10.0 g of tryptamine (1 eq.), 8.5 g (1 eq.) of 4-methoxybenzaldehyde, 5 g of 4A molecular sieves (as a dehydrating agent) and 200 ml of 1,2 - DCE. After dissolution, 2.85 g (0.4 eq.) of trifluoroacetic acid was added in one portion. The reaction mixture was brought to reflux. Completion of the reaction was monitored by TLC, after which the temperature was cooled to 30 °C, followed by the removal of molecular sieves through a loose glass wool plug. The solution was quenched with 150 ml of saturated NaHCO₃ aqueous solution and diluted with 500 ml of ethyl acetate. The organic layer was separated, washed with brine and dried with MgSO₄. After the solvent was removed, flash chromatography (10% CH₃OH / CH₂Cl₂) allowed isolation of the title product (12.8 g, 54%) as a solid.

### Example 4.2 [1-(4-Methoxy-phenyl)-1,3,4,9-tetrahydro-β-carbolin-2-yl]-phenyl-methanone

0.2 g of 1-(4-Methoxy-phenyl)-2,3,4,9-tetrahydro-1H-β-carboline (1eq.), 0.46 ml of N,N-diisopropylethylamine (3.7 eq.) and 3.2 mg of 4-dimethylaminopyridine (0.2 eq.) were dissolved with 8 ml of 1,2 - DCE in a scintillation vial. 0.113 g of Benzoyl chloride (1.12 eq.) was dissolved with 1 ml of 1,2 - DCE in another scintillation vial. These two solutions were mixed together, sealed and stirred overnight. Completion of the reaction was monitored by TLC, after which 2 ml of 10% citric acid aqueous solution was added to the reaction mixture. The solution was diluted with 50 ml of ethyl acetate. The organic layer was separated, washed twice with 10 ml of saturated NaHCO₃ aqueous solution, brine and dried with MgSO₄. After the solvent was removed, flash chromatography allowed isolation of the title product (0.21 g, 76%) as a solid.

### E. Synthesis schemes for other chemotype 4 compounds

### Compound with phenyl substitution

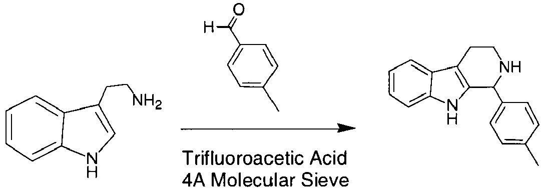

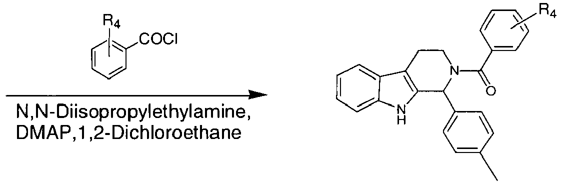

R₄ = *p*-Me, H, *p*-OMe, *p*-Cl, *p*-CF₃, *p*-F, *m*-Me, *m*-CF₃, *p*-Me, pentyl-F, *p*-NO₂, *p*-NMe₂, *p*-CN, *p,m*-DiCl, *p,o*-DiCl, *m,m*-DiCl, *o*-Me, *p*-Cl, *p*-^{t}Bu

### Compound with thiophene ring

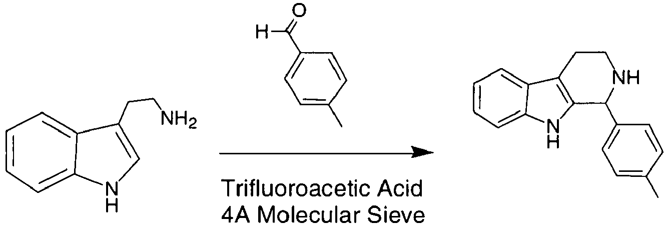

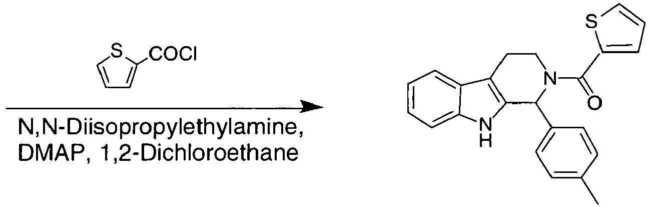

### Compound with cyclopentyl ring

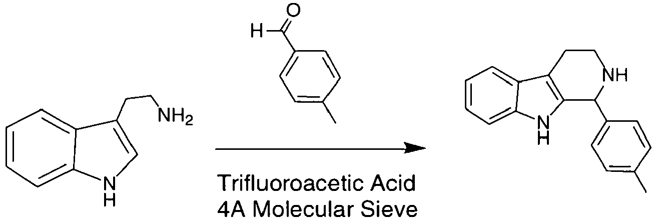

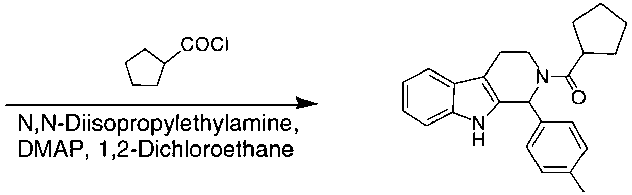

### Example 5 - p-Tolyl-(1-p-tolyl-1,3,4,9-tetrahydro-β-carbolin-2-yl)-methanone (compound 132C9)

### Example 5.1 - 1-p-Tolyl-2,3,4,9-tetrahydro-1H-β-carboline

A dry 250 ml round bottomed flask, equipped with a condenser and oil bath was charged with 5.0 g of tryptamine (1 eq.), 3.7 g (1 eq.) of *p*-tolualdehyde, 2.5 g of 4A molecular sieves (as a dehydrating agent) and 100 ml of 1,2 - DCE. After dissolution, 1 ml (0.4 eq.) of trifluoroacetic acid was added in one portion. The reaction mixture was brought to reflux. Completion of the reaction was monitored by TLC, after which the temperature was cooled to 30 °C, followed by the removal of molecular sieves through a loose glass wool plug. The solution was quenched with 50 ml of saturated NaHCO₃ aqueous solution and diluted with 200 ml of ethyl acetate. The organic layer was separated, washed with brine and dried with MgSO₄. After the solvent was removed, flash chromatography (10% CH₃OH / CH₂Cl₂) allowed isolation of the title product (7.6 g, 93%) as a solid.

### Example 5.2 -p-Tolyl-(1-p-tolyl-1,3,4,9-tetrahydro-β-carbolin-2-yl)-methanone

An oven-dried 250 ml round bottom flask, equipped with a stir bar was charged 1 g of 1-*p*-Tolyl-2,3,4,9-tetrahydro-1H-β-carboline (1eq.), 10 mg of 4-dimethylaminopyridine (catalyze amount) and 40 ml of 1,2 - DCE. The flask was sealed under Argon. Then 1.3 ml of N,N-diisopropylethylamine (2 eq.) and 0.62 g of *p*-toluoyl chloride (1.05 eq.) were sequentially added into reaction mixture. Completion of the reaction was monitored by TLC, after which 2 ml of THF and 10 ml of saturated NaHCO₃ aqueous solution were added. The stirring was continued for one more hour. The solution was diluted with 200 ml of ethyl acetate. The organic layer was separated, washed twice with 30 ml of saturated NaHCO₃ aqueous solution, brine and dried with NaSO₄. After the solvent was removed, flash chromatography allowed isolation of the title product (0.7 g, 49%) as a white solid.

### Example 6 - Effect of compounds on tumor cells

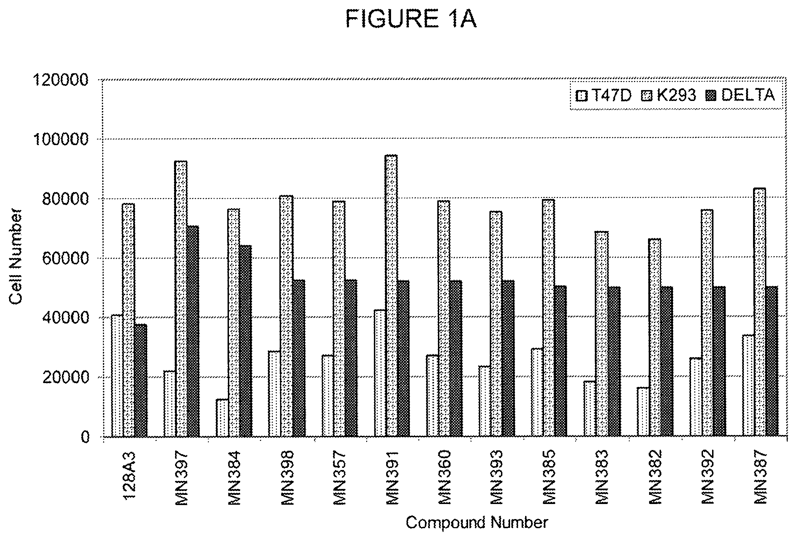

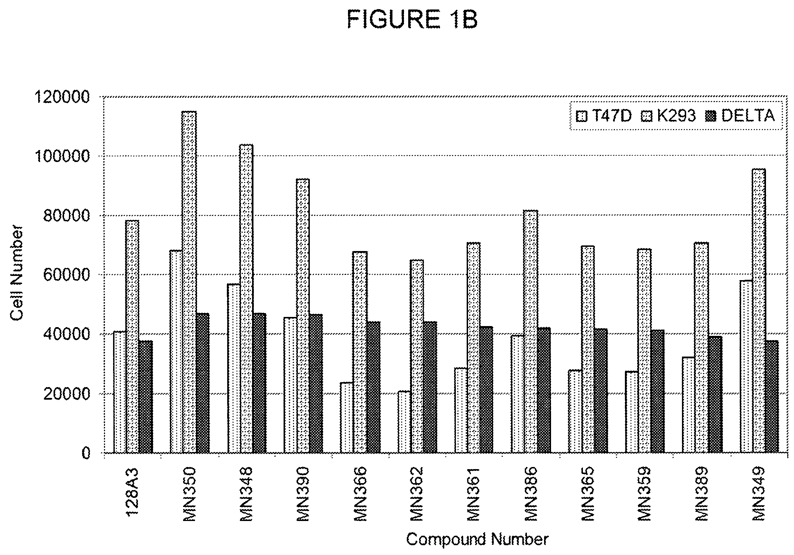

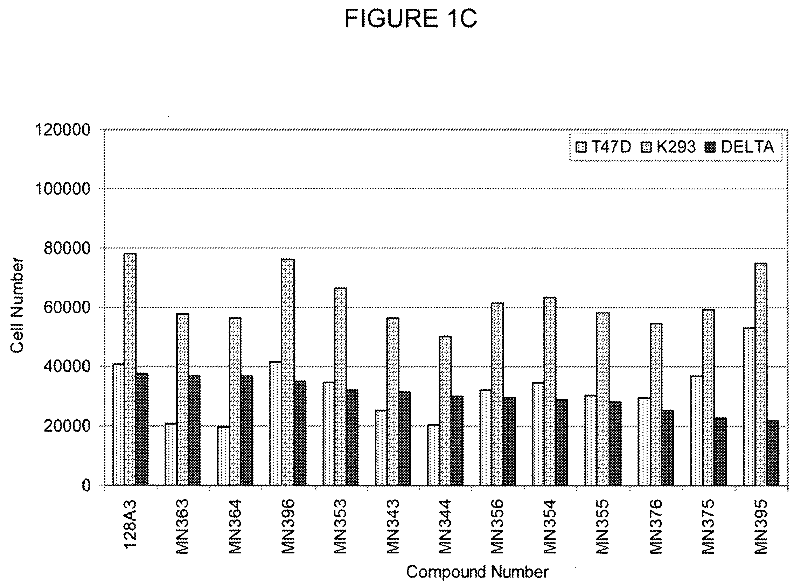

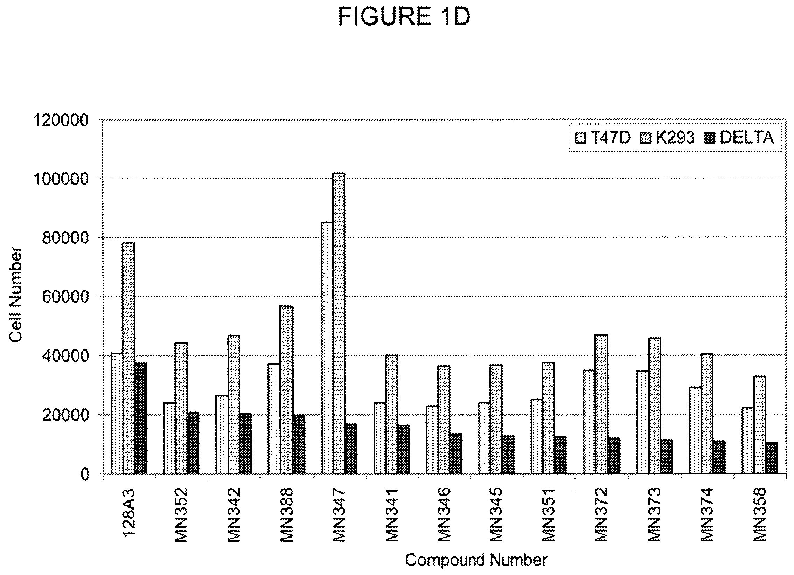

The following experimental procedure was used to obtain the results pictured in Figure 1A-D. HEK293 cells (MUC1-negative - ATCC HTB-133) or T47D cells (MUC1 and MUC1*-positive - ATCC CRL-1573) were plated at 20,000 cells/well in 96 well plates, at a final volume of 100 microliters. HEK293 cells were maintained and plated in DMEM medium with 10% serum, and T47D cells were maintained and plated in complete RPMI medium with 10% serum. Cells were plated in triplicate for each experimental condition. The day after plating, compounds were added at final concentrations of 0, 2.5µM, 5µM, and 10µM. This was done by adding 1 microliter of 250µM, 500µM, or 1mM stock concentrations of compounds. The zero compound control was the addition of 1 microliter of DMSO alone. These were prepared from 10mM stocks of compounds which were dissolved from their solid form in DMSO at room temperature. Solutions of compounds (250µM, 500µM, 1mM, or 10mM stocks) or DMSO for zero compound controls were stored at -20C. 48 hours after the addition of compounds to cells, cells were resuspended in 50 microliters of Trypsin-EDTA, and counted using a hemocytometer. To determine the effect of the test compound on the growth of MUC1-positive cells verses MUC1-negative cells, cell counts for each cell line were plotted, as was the difference, or "delta" between them (Delta = HEK293 cell counts minus T47D cell counts). Graphs were plotted using Microsoft Excel. Figures 1 A-D shows the effect of each compound at a final concentration of 5uM.

### Example 7 - The effect of various compounds on the growth of MUC1-positive cancer cells and MUC1-negative cells

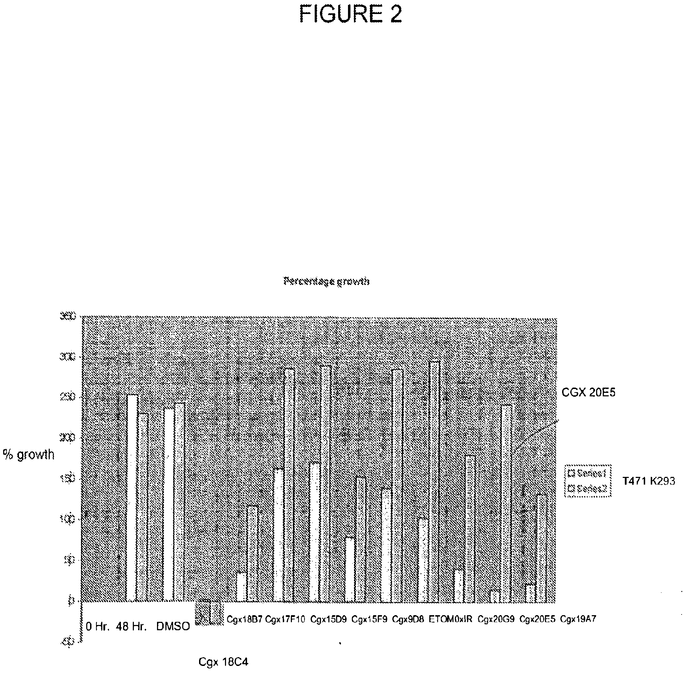

The bar graph shown in Figure 2 was drawn from results obtained using the following experimental procedure. This procedure is also the one that was used to obtain the percent growth numbers for compounds MN400-MN410, the chemical structures of which are shown in the application. HEK293 cells (also called K293, ATCC HTB-133) were plated at 5,000 cells/well, and T47D cells (ATCC CRL-1573) were plated at 10,000 cells/well in 96 well plates, at a final volume of 100 microliters. HEK293 cells were maintained and plated in DMEM medium with 10% serum, and T47D were maintained and plated in complete RPMI medium with 10% serum. Cells were plated in triplicate for each experimental condition. The day after plating, zero day counts were taken by resuspending cells in 50 microliters of Trypsin-EDTA and a hemocytometer. Then, compounds were added at a final concentration of 30µM. This was done by adding 1 microliter of 3mM stock concentration of compounds. Zero compound controls was the addition of 1 microliter of DMSO alone. Solutions of compounds or DMSO for zero compound controls were stored at - 20C. 48 hours after the addition of compounds to cells, cells were resuspended in 50 microliters of Trypsin-EDTA, and counted using a hemocytometer. Percent Normalized Growth was then calculated: Percent Normalized Growth = [(48 Hour cell counts with Compound Added) - (Zero-day cell counts)] / [(48 Hour Cell counts with DMSO added) - (Zero-day cell counts)] x 100%. Results were then plotted using Microsoft Excel.

### Example 8 - The effect of MUC1*-targeting compounds on the growth of MUC1-positive verses MUC1-negative cells

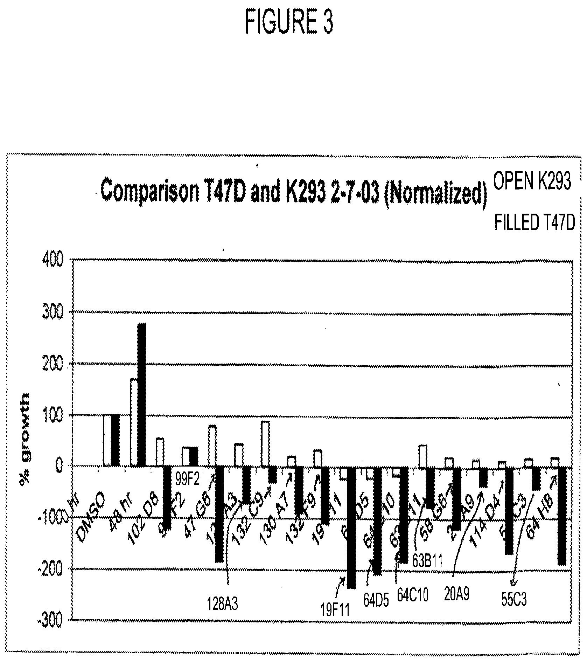

The bar graph shown in Figure 3 were the results of compound screening obtained using the following experimental procedure. HEK293 cells (also called K293, ATCC HTB-133) or T47D cells (ATCC CRL-1573) were plated at 3,000 cells/well in 96 well plates, at a final volume of 100 microliters. HEK293 cells were maintained and plated in DMEM medium with 10% serum, and T47D cells were maintained and plated in complete RPMI medium with 10% serum. Cells were plated in triplicate for each experimental condition. The day after plating, zero day counts were taken by resuspending cells in 50 microliters of Trypsin-EDTA and a hemocytometer. Then, compounds were added at a final concentration of 30µM. This was done by adding 1 microliter of 3mM stock concentration of compounds. Zero compound controls refer to the addition of either 1 microliter of DMSO alone, or no addition at all. Solutions of compounds or DMSO for zero compound controls were stored at -20C. 48 hours after the addition of compounds to cells, cells were resuspended in 50 microliters of Trypsin-EDTA, and counted using a hemocytometer. Percent Growth was then calculated: Percent Growth = (48 Hour cell counts with Compound Added) / (48 Hour Cell counts with DMSO alone added) x 100%. Results were then plotted using Microsoft Excel.

### Table 1: Peptide sequences (listed from N-terminus to C-terminus):

Full-length MUC1 Receptor (Mucin 1 precursor, Genbank Accession number: P15941)

MTPGTQSPFF LLLLLTVLTV VTGSGHASST PGGEKETSAT QRSSVPSSTE KNAVSMTSSV
LSSHSPGSGS STTQGQDVTL APATEPASGS AATWGQDVTS VPVTRPALGS TTPPAHDVTS
APDNKPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDTRPAPGS TAPPAHGVTS
APDTRPAPGS TAPPAHGVTS APDTRPAPGS TAPPAHGVTS APDNRPALGS TAPPVHNVTS
ASGSASGSAS TLVHNGTSAR ATTTPASKST PFSIPSHHSD TPTTLASHST KTDASSTHHS
SVPPLTSSNH STSPQLSTGV SFFFLSFHIS
NLQFNSSLED PSTDYYQELQ RDISEMFLQI YKQGGFLGLS NIKFRPGSVV VQLTLAFREG
TINVHDVETQ FNQYKTEAAS RYNLTISDVS VSDVPFPFSA QSGAGVPGWG IALLVLVCVL
VALAIVYLIA LAVCQCRRKN YGQLDIFPAR DTYHPMSEYP TYHTHGRYVP PSSTDRSPYE
KVSAGNGGSS LSYTNPAVAA ASANL (SEQ ID NO:1)

N-terminal MUC-1 signaling sequence for directing MUC1 receptor and truncated isoforms to cell membrane surface. Up to 3 amino acid residues may be absent at C-terminal end as indicated by variants in SEQ ID NOS:2, 3 and 4. MTPGTQSPFFLLLLLTVLT (SEQ ID NO:2). MTPGTQSPFFLLLLLTVLT VVTA (SEQ ID NO:3) MTPGTQSPFFLLLLLTVLT VVTG (SEQ ID NO:4)

A truncated MUC1 receptor isoform having nat-PSMGFR at its N-terminus and including the transmembrane and cytoplasmic sequences of a full-length MUC1 receptor ("nat-PSMGFRTC isoform" - An example of "PSMGFRTC" - shown excluding optional N-terminus signal sequence, which may be cleaved after translation and prior to expression of the receptor on the cell surface):

G TINVHDVETQ FNQYKTEAAS RYNLTISDVS VSDVPFPFSA QSGAGVPGWG IALLVLVCVL
VALAIVYLIA LAVCQCRRKN YGQLDIFPAR DTYHPMSEYP TYHTHGRYVP PSSTDRSPYE
KVSAGNGGSS LSYTNPAVAA ASANL
(SEQ ID NO:5)

A truncated MUC1 receptor isoform having nat-PSMGFR and PSIBR at its N-terminus and including the transmembrane and cytoplasmic sequences of a full-length MUC1 receptor ("CM isoform"- shown excluding optional N-terminus signal sequence, which may be cleaved after translation and prior to expression of the receptor on the cell surface):

GFLGLS NIKFRPGSVV VQLTLAFREG TINVHDVETQ FNQYKTEAAS RYNLTISDVS
VSDVPFPFSA QSGAGVPGWG IALLVLVCVL VALAIVYLIA LAVCQCRRKN YGQLDIFPAR
DTYHPMSEYP TYHTHGRYVP PSSTDRSPYE KVSAGNGGSS LSYTNPAVAA ASANL
(SEQ ID NO:6)

A truncated MUC1 receptor isoform having nat-PSMGFR + PSIBR + Unique Region at its N-terminus and including the transmembrane and cytoplasmic sequences of a full-length MUC1 receptor ("UR isoform"- shown excluding optional N-terminus signal sequences):

ATTTPASKST PFSIPSHHSD TPTTLASHST KTDASSTHHS TVPPLTSSNH
STSPQLSTGV SFFFLSFHIS NLQFNSSLED PSTDYYQELQ RDISEMFLQI
YKQGGFLGLS NIKFRPGSVV VQLTLAFREG TINVHDVETQ FNQYKTEAAS
RYNLTISDVS VSDVPFPFSA QSGAGVPGWG IALLVLVCVL VALAIVYLIA
LAVCQCRRKN YGQLDIFPAR DTYHPMSEYP TYHTHGRYVP PSSTDRSPYE KVSAGNGGSS
LSYTNPAVAA ASANL (SEQ ID NO:7)

A truncated MUC1 receptor isoform including the transmembrane and cytoplasmic sequences of a full-length MUC1 receptor ("Y isoform"- shown excluding optional N-terminus signal sequence, which may be cleaved after translation and prior to expression of the receptor on the cell surface):

GSGHASSTPG GEKETSATQR SSVPSSTEKN AFNSSLEDPS TDYYQELQRD ISEMFLQIYK
QGGFLGLSNI KFRPGSVVVQ LTLAFREGTI NVHDMETQFN QYKTEAASRY NLTISDVSVS
DVPFPFSAQS GAGVPGWGIA LLVLVCVLVA LAIVYLIALA VCQCRRKNYG QLDIFPARDT
YHPMSEYPTY HTHGRYVPPS STDRSPYEKV SAGNGGSSLS YTNPAVAATS ANL
(SEQ ID NO:8)

A truncated MUC1 receptor isoform having nat-PSMGFR + PSIBR + Unique Region + Repeats at its N-terminus and including the transmembrane and cytoplasmic sequences of a full-length MUC1 receptor ("Rep isoform"- shown excluding optional N-terminus signal sequence, which may be cleaved after translation and prior to expression of the receptor on the cell surface):

LDPRVRTSAP DTRPAPGSTA PQAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DTRPAPGSTA PPAHGVTSAP DTRPAPGSTA PPAHGVTSAP DTRPAPGSTA
PPAHGVTSAP DNRPALGSTA PPVHNVTSAS GSASGSASTL VHNGTSARAT TTPASKSTPF
SIPSHHSDTP TTLASHSTKT DASSTHHSSV PPLTSSNHST SPQLSTGVSF FFLSFHISNL
QFNSSLEDPS TDYYQELQRD ISEMFLQIYK QGGFLGLSNI KFRPGSVVVQ LTLAFREGTI
NVHDVETQFN QYKTEAASRY NLTISDVSVS DVPFPFSAQS GAGVPGWGIA LLVLVCVLVA
LAIVYLIALA VCQCRRKNYG QLDIFPARDT YHPMSEYPTY HTHGRYVPPS STDRSPYEKV
SAGNGGSSLS YTNPAVAAAS ANL
(SEQ ID NO:9)

Native Primary Sequence of the MUC1 Growth Factor Receptor (nat-PSMGFR - an example of "PSMGFR"): GTINVHDVETQFNQYKTEAA**SRY**NLTISDVSVSDVPFPFSAQSGA (SEQ ID NO:10)

Native Primary Sequence of the MUC1 Growth Factor Receptor (nat-PSMGFR - An example of "PSMGFR"), having a single amino acid deletion at the N-terminus of SEQ ID NO: 10): TINVHDVETQFNQYKTEAA**SRY**NLTISDVSVSDVPFPFSAQSGA (SEQ ID NO:11)

"SPY" functional variant of the native Primary Sequence of the MUC1 Growth Factor Receptor having enhanced stability (var-PSMGFR - An example of "PSMGFR"): GTINVHDVETQFNQYKTEAA**SPY**NLTISDVSVSDVPFPFSAQSGA (SEQ ID NO:12)

"SPY" functional variant of the native Primary Sequence of the MUC1 Growth Factor Receptor having enhanced stability (var-PSMGFR - An example of "PSMGFR"), having a single amino acid deletion at the C-terminus of SEQ ID NO:12): TINVHDVETQFNQYKTEAA**SPY**NLTISDVSVSDVPFPFSAQSGA (SEQ ID NO:13)

Truncated PSMGFR receptor (TR) (having "SPY" sequence of var-PSMGFR): GTINVHDVETQFNQYKTEAA**SPY**NLTISDVSVS (SEQ ID NO:14)

Extended Sequence of MUC1 Growth Factor Receptor (ESMGFR) (having "SPY" sequence of var-PSMGFR): VQLTLAFREGTINVHDVETQFNQYKTEAA**SPY**NLTISDVSVSDVPFPF (SEQ ID NO:15)

Tumor-Specific Extended Sequence of MUC1 Growth Factor Receptor (TSESMGFR) (having "SPY" sequence of var-PSMGFR): SVVVQLTLAFREGTINVHDVETQFNQYKTEAA**SPY**NLTISDVSVS DVPFPFSAQSGA (SEQ ID NO:16)

Primary Sequence of the Interchain Binding Region) (PSIBR): GFLGLSNIKFRPGSVVVQLTLAFRE (SEQ ID NO:17)

Truncated Interchain Binding Region) (TPSIBR): SVVVQLTLAFREG (SEQ ID NO:18)

Repeat Motif 2 (RM2): PDTRPAPGSTAPPAHGVTSAPDTRPAPGSTAPPAHGVTSA (SEQ ID NO: 19)

## Claims

1. A compound comprised of the formula: 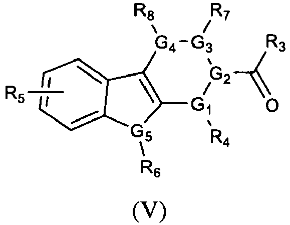 or a steroisomer, tautomer, pharmaceutically acceptable salt or ester thereof, for use in treating or preventing cancer **characterised by** aberrant expression of MUC1, wherein the treatment or prevention comprises a determination of whether the subject's cancer aberrantly expresses MUC1 and treating the subject with the compound if so, wherein,
R3 is selected from 2-thiofuranyl, phenyl optionally substituted with one or more of the following substituents:
H,
3-, or 4-Cl,
4-F,
2, 3, 4 ,5, 6-per-fluoro,
2, 4-di-Cl,
3, 5-di-Cl,
3 ,4-di-Cl,
4-OCH₃,
3-, or 4-CF₃,
4-CN,
2-, 3-, 4-Me,
2, 3-di-Me,
4-t-Bu,
4-NO₂, and
4-N(CH₃)₂;
R4 is phenyl ring optionally substituted with H, 4-OCH₃, 4-Cl, or 4-CF₃;
R5 is H;
R6 is H;
R7 is H;
R8 is H;
G1 is CH;
G2 is N;
G3 is CH;
G4 is CH; and
G5 is N.

2. A compound for use according to claim 1, wherein the compound has the structure of formula: 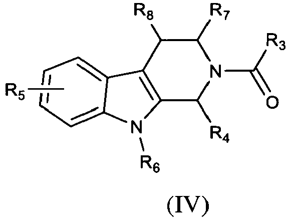 in which R3, R4, R5, R6, R7 and R8 are as defined in claim 1.

3. A compound for use according to claim 1, wherein the compound has the structure of formula: 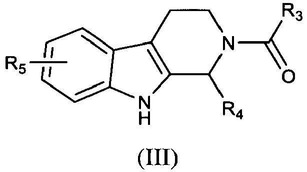 in which R3, R4 and R5 are as defined in claim 1.

4. A compound for use according to claim 1 wherein, the compound has the structure of formula: 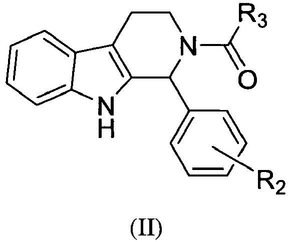 in which R3 is as defined in claim 1 and R2 has the same definition as R4 in claim 1.

5. A compound for use as defined in any one of the preceding claims, for use as a metal chelator.

6. A compound for use according to claim 1, wherein the compound is MN341, MN342, MN344, MN345, MN346, MN347, MN348, MN349, MN350, MN351, MN352, MN353, MN354, MN355, MN356, MN357, MN358, MN359, MN361, MN362, MN363, MN364, MN365, MN366, MN372, MN373, MN374, MN375, MN376, MN382, MN383, MN384, MN385, MN386, MN387, MN388, MN389, MN390, MN391, MN392, MN395, MN396, MN397 or MN398.

## Patentansprüche

1. Verbindung, die in der folgenden Formel besteht: 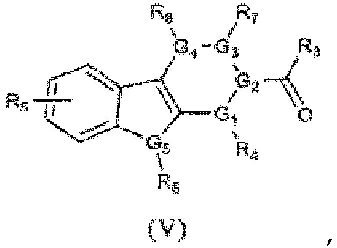 oder ein Stereoisomer, Tautomer, pharmazeutisch akzeptables Salz oder Ester davon zur Verwendung beim Behandeln oder Verhindern einer Krebserkrankung, die durch eine aberrante Expression von MUC1 gekennzeichnet ist, wobei die Behandlung oder Verhinderung eine Bestimmung, ob die Krebserkrankung der Person MUC1 aberrant exprimiert, und das Behandeln der Person mit der Verbindung, falls dies der Fall ist, umfasst, wobei
R3 aus 2-Thiofuranyl und Phenyl, das gegebenenfalls mit einem oder mehreren der folgenden Substituenten substituiert ist, ausgewählt ist:
H,
3- oder 4-Cl,
4-F,
2, 3, 4, 5, 6-Perfluoro,
2,4-Di-Cl,
3,5-Di-Cl,
3,4-Di-Cl,
4-OCH3,
3- oder 4-CF₃,
4-CN,
2-, 3-, 4-Me,
2,3-Di-Me,
4-t-Bu,
4-NO₂ und
4-N(CH₃)₂;
R4 ein Phenylring ist, der gegebenenfalls mit H, 4-OCH₃, 4-Cl oder 4-CF₃ substituiert ist;
R5 H ist;
R6 H ist;
R7 H ist;
R8 H ist;
G1 CH ist;
G2 N ist;
G3 CH ist;
G4 CH ist und
G5 N ist.

2. Verbindung zur Verwendung nach Anspruch 1, wobei die Verbindung die Struktur der folgenden Formel aufweist: 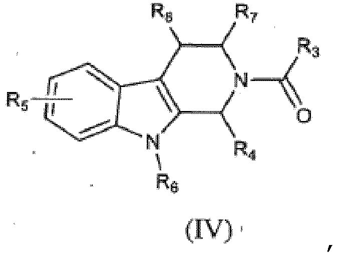 in der R3, R4, R5, R6, R7 und R8 wie in Anspruch 1 definiert sind.

3. Verbindung zur Verwendung nach Anspruch 1, wobei die Verbindung die Struktur der folgenden Formel aufweist: 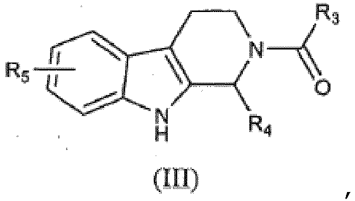 in der R3, R4 und R5 wie in Anspruch 1 definiert sind.

4. Verbindung zur Verwendung nach Anspruch 1, wobei die Verbindung die Struktur der folgenden Formel aufweist: 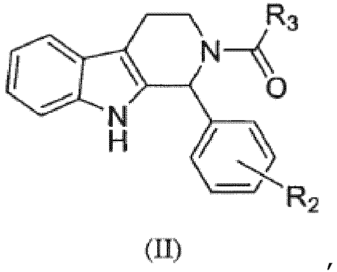 in der R3 wie in Anspruch 1 definiert ist und R2 dieselbe Definition wie R4 in Anspruch 1 hat.

5. Verbindung zur Verwendung wie in einem der vorhergehenden Ansprüche definiert zur Verwendung als ein Metallchelatbildner.

6. Verbindung zur Verwendung nach Anspruch 1, wobei die Verbindung MN341, MN342, MN344, MN345, MN346, MN347, MN348, MN349, MN350, MN351, MN352, MN353, MN354, MN355, MN356, MN357, MN358, MN359, MN361, MN362, MN363, MN364, MN365, MN366, MN372, MN373, MN374, MN375, MN376, MN382, MN383, MN384, MN385, MN386, MN387, MN388, MN389, MN390, MN391, MN392, MN395, MN396, MN397 oder MN398 ist.

## Revendications

1. Composé constitué de la formule : 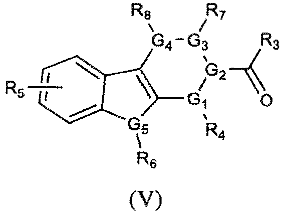 ou stéréoisomère, tautomère, sel pharmaceutiquement acceptable ou ester de celui-ci, destiné à être utilisé en traitement ou prévention d'un cancer **caractérisé par** l'expression aberrante de MUC1, le traitement ou la prévention comprenant le fait de déterminer si le cancer du sujet exprime MUC1 de façon aberrante et le traitement du sujet avec le composé si c'est le cas, dans lequel,
R3 est choisi entre les groupes 2-thiofuranyle et phényle éventuellement substitué par un ou plusieurs des substituants suivants :
H,
3-Cl ou 4-Cl,
4-F,
2,3,4,5,6-perfluoro,
2,4-di-Cl,
3,5-di-Cl
3,4-di-Cl,
4-OCH₃,
3-CF₃ ou 4-CF₃,
4-CN,
2-Me, 3-Me, 4-Me,
2,3-di-Me,
4-t-Bu,
4-NO₂ et
4-N(CH₃)₂ ;
R4 est un noyau phényle éventuellement substitué par H, 4-OCH₃, 4-Cl ou 4-CF₃ ;
R5 est H ;
R6 est H ;
R7 est H ;
R8 est H ;
G1 est CH ;
G2 est N ;
G3 est CH ;
G4 est CH ; et
G5 est N.

2. Composé destiné à être utilisé selon la revendication 1, le composé ayant la structure de formule : 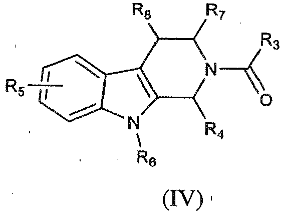 dans laquelle R3, R4, R5, R6, R7 et R8 sont tels que définis dans la revendication 1.

3. Composé destiné à être utilisé selon la revendication 1, le composé ayant la structure de formule : 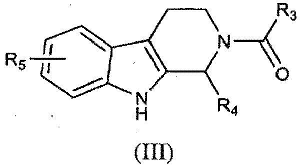 dans laquelle R3, R4 et R5 sont tels que définis dans la revendication 1.

4. Composé destiné à être utilisé selon la revendication 1, le composé ayant la structure de formule : 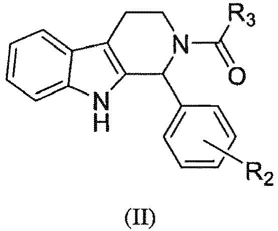 dans laquelle R3 est tel que défini dans la revendication 1 et R2 a la même définition que R4 dans la revendication 1.

5. Composé destiné à être utilisé comme défini dans l'une quelconque des revendications précédentes, destiné à être utilisé comme chélateur de métaux.

6. Composé destiné à être utilisé selon la revendication 1, le composé étant MN341, MN342, MN344, MN345, MN346, MN347, MN348, MN349, MN350, MN351, MN352, MN353, MN354, MN355, MN356, MN357, MN358, MN359, MN361, MN362, MN363, MN364, MN365, MN366, MN372, MN373, MN374, MN375, MN376, MN382, MN383, MN384, MN385, MN386, MN387, MN388, MN389, MN390, MN391, MN392, MN395, MN396, MN397 ou MN398.
